# EUROPEAN PATENT APPLICATION

(11) **EP 0 950 418 A2**
(43) Date of publication of application: **20.10.1999**
(21) Application number: 99107184.6
(22) Date of filing: 13.04.1999
(51) Int. Cl.: A61K 45/06, A61K 38/12

(54) **Composition for preventing or treating ischemic disease**

(30) Priority: 14.04.1998 JP 10314198
(71) Applicant: Takeda Chemical Industries, Ltd., Osaka 541-0045 (JP)
(72) Inventor: Watanabe, Toshifumi, Osaka 586-0092 (JP); Shiraishi, Mitsuru, Hyogo 661-0002 (JP); Wakimasu, Mitsuhiro, Hyogo 666-0166 (JP)
(74) Representative: Winter, Brandl & Partner

(57) **Abstract**

The present invention is to provide a pharmaceutical composition for preventing or treating ischemic disease comprising a compound having endothelin antagonistic activity or a salt thereof in combination with a compound having activity for inhibiting Na-H exchange or a salt thereof, which shows remarkable prophylactic or therapeutic effect on ischemic disease and which serves to decrease remarkably the dosage of the individual effective components, and as a result, suppresses undesirable side effects observed in the case of administering the respective compounds singly.

## Description

### Technical Field of the Invention

The present invention relates to a pharmaceutical composition for preventing or treating ischemic disease comprising a compound having endothelin antagonistic activity or a salt thereof in combination with a compound having activity for inhibiting Na-H exchange or a salt thereof.

### Prior Art

Endothelin (ET), a vasoconstrictive peptide comprising 21 amino acids, was isolated from the culture supernatant of the endothelial cells of porcine aortas and structurally determined by Yanagisawa et al. in 1988 [Yanagisawa et al., Nature, 332, 411 (1988)]. It is reported that endothelin has not only vasoconstrictive action but also various physiological activities such as cardiac stimulating activity, renal mesangial contractive activity, etc., and also the presence of at least 3 isoforms (ET-1, ET-2, ET-3) and 2 receptors (ET_{A}, ET_{B}) is reported.

Since the development of endothelin, there have been intensive investigations in search of endothelin antagonists that antagonize endothelin on endothelin receptors and inhibit the activity of endothelin, with the aim of developing therapeutic agents for diseases caused by endothelin. As a result, compounds having endothelin antagonistic activity are disclosed in EP-A-552489, EP-A-528312, EP-A-499266, WO91/13089, EP-A-436189, EP-A-457195, EP-A-510526, WO92/12991, Japanese Patent Unexamined Publication No. 288099/1992, Japanese Patent Unexamined Publication No. 244097/1992, Japanese Patent Unexamined Publication No. 261198/1992, EP-A-496452, EP-A-526708, EP-A-526642, EP-A-510526, EP-A-460679, WO92/20706, EP-A-626174, EP-A-655463, EP-A-714909, Japanese Patent Unexamined Publication No. 173161/1995, etc. These endothelin antagonists are suggested to be useful for the prevention or treatment of hypertension, cardiovascular or cerebrovascular disease, renal disease, asthma, inflammation, arthritis, etc..

On the other hand, Na-H exchange inhibitors, assumed to exhibit ameliorating and cell-protecting action in cell disorders under ischemic conditions, especially on the myocardium, are drawing attention in the field of therapeutic drugs for ischemic diseases.

Amiloride, an acylguanidine derivative and potassium-retaining diuretic, possesses weak activities for inhibiting Na-H exchange and potent activities for inhibiting sodium channel.

As Na-H exchange inhibitors, various kinds of acylguanidine derivatives are disclosed in Japanese Patent Unexamined Publication No. 228082/1994, WO 96/04241, EP 708091 and EP 708088, etc.

### Detailed Description of the Invention

The present invention is intended, by using a compound having endothelin antagonistic activity or a salt thereof in combination with a compound having activity for inhibiting Na-H exchange or a salt thereof, to perform especially remarkable effects in the prevention or treatment of ischemic diseases, especially ischemic cardiac disease, and in particular, myocardial infarction, cardiac insufficiency, arrhythmia, etc., and to cover up defects observed in administration of a medicine consisting of a single component.

In view of the above-mentioned circumstances, the present inventors have actually combined, for the first time, a compound having endothelin antagonistic activity or a salt thereof with a compound having activity for inhibiting Na-H exchange or a salt thereof, and as a result, they have found that the co-use performs especially remarkable effects (e.g. treatment effect, safety, stability, dose, administration route, method of use, etc.) which were not observed in the administration of the respective compounds singly. The present inventors made further investigations based on this finding, and developed the present invention.

More specifically, the present invention relates to
(1) a pharmaceutical composition for treating ischemic disease comprising a compound having endothelin antagonistic activity or a salt thereof in combination with a compound having activity for inhibiting Na-H exchange or a salt thereof;
(2) a composition of the above (1), which is for the prevention or treatment of ischemic heart disease (ischemic cardiac disease);
(3) a composition of the above (1), which is for the prevention or treatment of ischemic encephalic disease;
(4) a composition of the above (1), which is for the prevention or treatment of ischemic renal disease;
(5) a composition of the above (1), which is for the prevention or treatment of myocardial infarction;
(6) a composition of the above (1), which is for the prevention or treatment of arrhythmia;
(7) a composition of the above (1), which is for the prevention or treatment of congestive heart failure (cardiac insufficiency);
(8) a composition of the above (1), wherein the compound having endothelin antagonistic activity or a salt thereof is Cyclo[-D-Asp-Asp(R1)-Asp-D-Thg(2)-Leu-D-Trp-], in which Asp(R1) is aspartic acid β-4-phenylpiperazineamide residue and Thg(2) is 2-thienylglycine residue, or a salt thereof;
(9) a composition of the above (1), wherein the compound having activity for inhibiting Na-H exchange or a salt thereof is cariporide;
(10) a composition of the above (1), wherein the compound having activity for inhibiting Na-H exchange or a salt thereof is a compound of the formula: 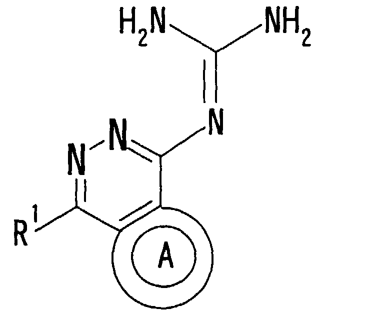 wherein the ring A is an optionally substituted benzene ring or an optionally nitrogen-containing 6-membered aromatic ring and R¹ is an optionally substituted aromatic ring group, or a salt thereof;
(11) a composition of the above (1), wherein the compound having activity for inhibiting Na-H exchange or a salt thereof is a compound of the formula: 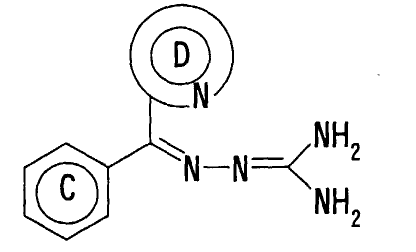 wherein the ring C is an optionally substituted benzene ring, and the ring D is an optionally substituted nitrogen-containing 6-membered aromatic ring, or a salt thereof;
(12) a composition of the above (1), wherein the compound having activity for inhibiting Na-H exchange or a salt thereof is a compound of the formula: 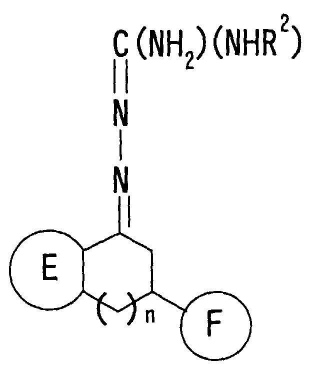 wherein the ring E is an optionally substituted 5- to 6-membered aromatic heterocyclic ring, the ring F is an optionally substituted 5- to 6-membered aromatic homocyclic ring or an optionally substituted 5- to 6-membered aromatic heterocyclic ring, R² is a hydrogen atom, a hydroxy group or a lower alkyl group and n is 0 or 1, or a salt thereof;
(13) a pharmaceutical composition for treating ischemic disease comprising disodium salt of Cyclo[-D-Asp-Asp(R1)-Asp-D-Thg(2)-Leu-D-Trp-], in which Asp(R1) is aspartic acid β-4-phenylpiperazineamide residue and Thg(2) is 2-thienylglycine residue, in combination with cariporide; etc.

Examples of the compound having endothelin antagonistic activity or a salt thereof to be used in the present invention include, for example, compounds described in EP-A-552489, EP-A-528312, EP-A-499266, WO91/13089, EP-A-436189, EP-A-457195, EP-A-510526, WO92/12991, Japanese Patent Unexamined Publication No. 288099/1992, Japanese Patent Unexamined Publication No. 244097/1992, Japanese Patent Unexamined Publication No. 261198/1992, EP-A-496452, EP-A-526708, EP-A-526642, EP-A-510526, EP-A-460679, WO92/20706, EP-A-626174, EP-A-655463, EP-A-714909, Japanese Patent Unexamined Publication No. 1995(H7)-173161, etc. Among others, the following compounds are preferably employed: disodium salt of Cyclo[-D-Asp-Asp(R1)-Asp-D-Thg(2)-Leu-D-Trp-], in which Asp(R1) is aspartic acid β-4-phenylpiperazineamide residue and Thg(2) is 2-thienylglycine residue (hereinbelow, referred to as Compound A);

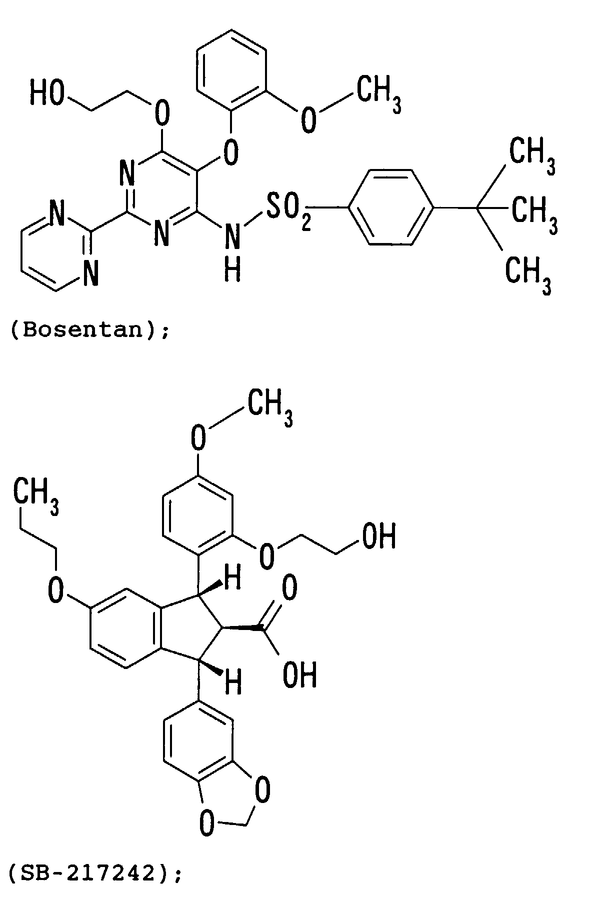

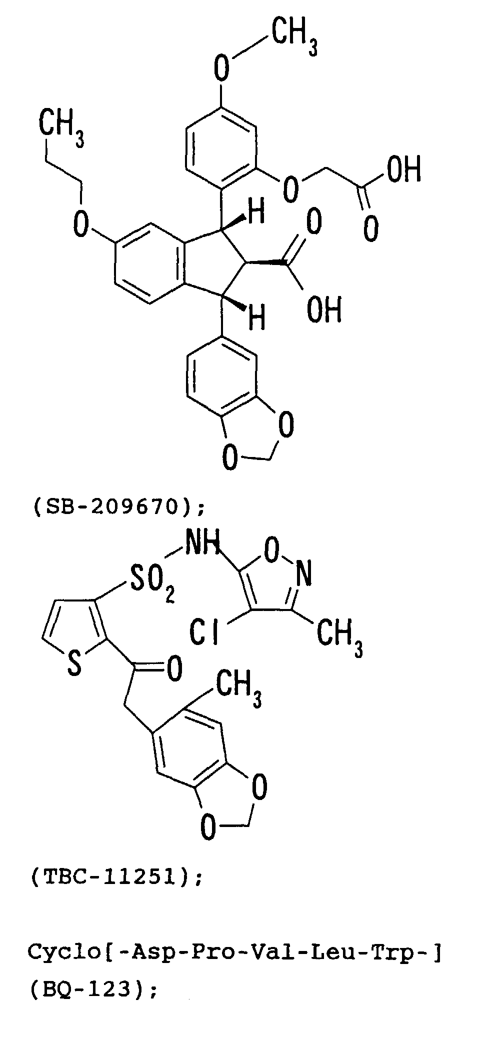

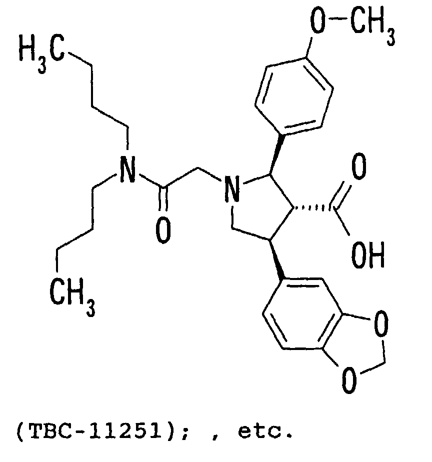

Among others, Cyclo[-D-Asp-Asp(R1)-Asp-D-Thg(2)-Leu-D-Trp-] in which Asp(R1) is aspartic acid β-4-phenylpiperazineamide residue and Thg(2) is 2-thienylglycine residue, or a salt thereof is preferable, and in particular, 2Na salt of Cyclo[-D-Asp-Asp(R1)-Asp-D-Thg(2)-Leu-D-Trp-], in which Asp(R1) is aspartic acid β-4-phenylpiperazineamide residue and Thg(2) is 2-thienylglycine residue, is preferable.

Examples of the compound having activity for inhibiting Na-H exchange or a salt thereof, include, for example, compounds described in EP-A-708091, EP-A-708088, WO96/04241, Japanese Patent Unexamined Publication No. 1994(H6)-228082, Japanese Patent Application No. 1997(H9)-224945 (Japanese Patent Unexamined Publication No. 1998(H10)-114753), Japanese Patent Application No. 1997(H9)-224946 (Japanese Patent Unexamined Publication No. 1998(H10)-114744), Japanese Patent Application No. 1998(H10)-38720 (PCT/JP99/00703), etc. Among others, the following compounds are preferably employed: 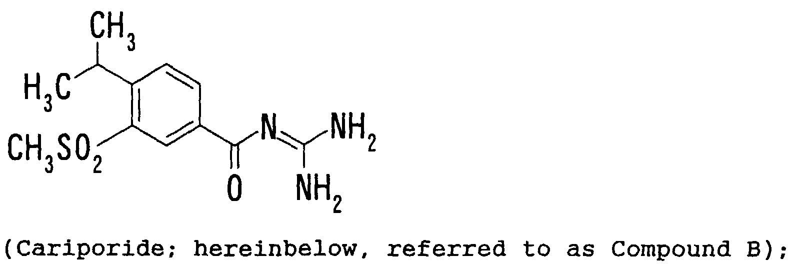
a compound of the formula: (I) 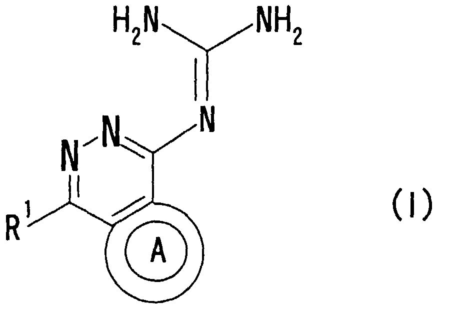 wherein the ring A is an optionally substituted benzene ring or an optionally substituted nitrogen-containing 6-membered aromatic ring, and R¹ is an optionally substituted aromatic ring group, or a salt thereof;
a compound of the formula: (II) 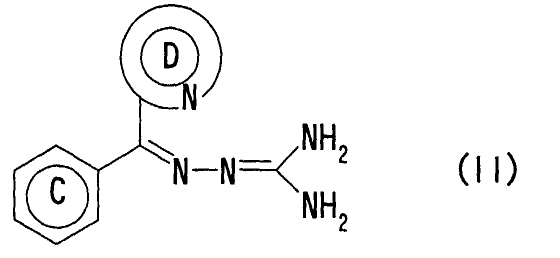 wherein the ring C is an optionally substituted benzene ring and the ring D is an optionally substituted nitrogen-containing 6-membered aromatic ring, or a salt thereof;
a compound of the formula: (III) 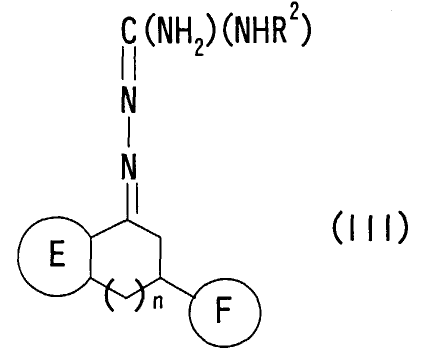 wherein the ring E is an optionally substituted 5- to 6-membered aromatic heterocyclic ring, the ring F an optionally substituted 5- to 6-membered aromatic homocyclic ring or an optionally substituted 5- to 6-membered aromatic heterocyclic ring, R² is a hydrogen atom, a hydroxy group or a lower alkyl group, and n is 0 or 1, or a salt thereof; etc.

In the above formula (I), the ring A is an optionally substituted benzene ring or an optionally substituted nitrogen-containing 6-membered aromatic ring.

Examples of the nitrogen-containing 6-membered aromatic ring represented by the ring A include, for example, a pyridine ring, a pyrazine ring, a pyridazine ring, etc.

Here, the ring A is condensed with the 4- and 5-positions (side d) of the pyridazine ring having a guanidyl group at the 3-position; for example, the pyridine ring is condensed at the 2- and 3-positions (side b) or at the 3- and

4-positions (side c), the pyrazine ring is condensed at the 2- and 3-positions (side b), and the pyridazine ring is condensed at the 3- and 4-positions (side c) or at the 4- and 5-positions (side d).

The benzene ring or nitrogen-containing 6-membered aromatic ring represented by the ring A may be substituted with 1 to 4 (preferably 1 or 2) identical or different substituents selected from, for example, (1) a halogen atom, (2) a hydroxy group, (3) a nitro group, (4) a cyano group, (5) a lower alkyl group optionally substituted with a halogen atom, (6) a lower alkoxy group optionally substituted with a halogen atom, (7) a lower acyl group, (8) a mercapto group optionally substituted with a lower alkyl group, (9) an optionally substituted amino group and (10) an optionally substituted phenyl group, at any possible position.

Examples of the halogen atom as (1) include, for example, chlorine, bromine, fluorine, iodine, etc.

Examples of the lower alkyl group optionally substituted with a halogen atom (for example, chlorine, bromine, fluorine, iodine, etc.) as (5) include, for example, a C₁₋₆ alkyl group (for example, methyl, ethyl, propyl, isopropyl, butyl, isobutyl, s-butyl, t-butyl, pentyl, hexyl, etc.), a halogeno C₁₋₆ alkyl group (for example, CF₃, CF₂CF₃, CH₂F, CHF₂), etc.

Examples of the lower alkoxy group optionally substituted with a halogen atom (for example, chlorine, bromine, fluorine, iodine, etc.) as (6) include, for example, a C₁₋₆ alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno C₁₋₆ alkoxy group (for example, CF₃O, CHF₂O, etc.), etc..

Examples of the lower acyl group as (7) include, for example, a lower acyl group derived from a carboxylic acid, a sulfinic acid, a sulfonic acid, etc.

Here, examples of the lower acyl group derived from a carboxylic acid include, for example, formyl, a lower (C₁₋₆) alkyl-carbonyl (C₁₋₆ alkanoyl) (for example, acetyl, propionyl, butyryl, isobutyryl, valeryl, pivaloyl, etc.), a C₃₋₆ cycloalkyl-carbonyl (for example, cyclopropylcarbonyl, cyclobutylcarbonyl, cyclopentylcarbonyl, cyclohexylcarbonyl, etc.), benzoyl, etc.

Examples of the lower acyl group derived from a sulfinic acid include, for example, a lower (C₁₋₆) alkylsulfinyl (for example, methylsulfinyl, ethylsulfinyl, propylsulfinyl, isopropylsulfinyl, butylsulfinyl, isobutylsulfinyl, s-butylsulfinyl, t-butylsulfinyl, pentylsulfinyl, hexylsulfinyl, etc.), a C₃₋₆ cycloalkylsulfinyl (for example, cyclopropylsulfinyl, cyclobutylsulfinyl, cyclopentylsulfinyl, cyclohexylsulfinyl, etc.), phenylsulfinyl, etc.

Examples of the lower acyl group derived from a sulfonic acid include, for example, a lower (C₁₋₆) alkylsulfonyl (for example, methylsulfonyl, ethylsulfonyl, propylsulfonyl, isopropylsulfonyl, butylsulfonyl, isobutylsulfonyl, t-butylsulfonyl, pentylsulfonyl, hexylsulfonyl, etc.), a C₃₋₆ cycloalkylsulfonyl (for example, cyclopropylsulfonyl, cyclobutylsulfonyl, cyclopentylsulfonyl, cyclohexylsulfonyl, etc.), phenylsulfonyl, etc.

Examples of the mercapto group optionally substituted with a lower alkyl group (for example, a C₁₋₆ alkyl such as methyl, ethyl, propyl, isopropyl, butyl, isobutyl, t-butyl, pentyl, hexyl, etc.) as (8) include, for example, amercapto group, a C₁₋₆ alkylthiogroup (for example, methylthio, ethylthio, propylthio, isopropylthio, butylthio, isobutylthio, t-butylthio, pentylthio, hexylthio, etc.), etc.

Examples of the optionally substituted amino group as (9) include, for example, amino groups which may be substituted with 1 or 2 identical or different substituents selected from lower (C₁₋₆) alkyl (for example, methyl, ethyl, propyl, isopropyl, butyl, isobutyl, s-butyl, t-butyl, pentyl, hexyl, etc.), lower (C₁₋₆) alkoxy (for example, methoxy, ethoxy, propoxy, isopropoxy, butoxy, isobutoxy, s-butoxy, t-butoxy, pentyloxy, hexyloxy, etc.), halogeno lower (C₁₋₆) alkyl (for example, CF₃, CF₃CF₂, CH₂F, CHF₂, etc.), C₃₋₆ cycloalkyl (for example, cyclopropyl, cyclobutyl, cyclopentyl, cyclohexyl, etc.), hydroxy group, carbamoyl, phenyl, phenyl-C₁₋₆ alkyl (for example, benzyl, phenethyl, 3-phenylpropyl, 4-phenylbutyl, etc.), formyl, lower (C₁₋₆) alkanoyl (for example, acetyl, propionyl, butyryl, isobutyryl, valeryl, pivaloyl, etc.), C₃₋₆ cycloalkylcarbonyl (for example, cyclopropylcarbonyl, cyclobutylcarbonyl, cyclopentylcarbonyl, cyclohexylcarbonyl, etc.), benzoyl, phenyl-C₂₋₄ alkanoyl (for example, phenylacetyl, phenylpropionyl, etc.), lower (C₁₋₆) alkoxy-carbonyl (for example, methoxycarbonyl, ethoxycarbonyl, propoxycarbonyl, isopropoxycarbonyl, butoxycarbonyl, isobutoxycarbonyl, s-butoxycarbonyl, t-butoxycarbonyl, pentyloxycarbonyl, hexyloxycarbonyl, etc.), phenoxycarbonyl, phenyl-C₁₋₄ alkoxy-carbonyl (for example, benzyloxycarbonyl, phenylethoxycarbonyl, etc.), lower (C₁₋₆) alkylsulfinyl (for example, methylsulfinyl, ethylsulfinyl, propylsulfinyl, isopropylsulfinyl, butylsulfinyl, isobutylsulfinyl, s-butylsulfinyl, t-butylsulfinyl, pentylsulfinyl, hexylsulfinyl, etc.), C₃₋₆ cycloalkylsulfinyl (for example, cyclopropylsulfinyl, cyclobutylsulfinyl, cyclopentylsulfinyl, cyclohexylsulfinyl, etc.), phenylsulfinyl, lower (C₁₋₆) alkylsulfonyl (for example, methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, hexylsulfonyl, etc.), C₃₋₆ cycloalkylsulfonyl (for example, cyclopropylsulfonyl, cyclobutylsulfonyl, cyclopentylsulfonyl, cyclohexylsulfonyl, etc.), lower (C₁₋₆) alkoxysulfonyl (for example, methoxysulfonyl, ethoxysulfonyl, propoxysulfonyl, isopropoxysulfonyl, butoxysulfonyl, isobutoxysulfonyl, s-butoxysulfonyl, t-butoxysulfonyl, pentyloxysulfonyl, hexyloxysulfonyl, etc.), phenylsulfonyl, etc.

Also, two of the substituents may form a cyclic amino group in cooperation with a nitrogen atom, and examples of the cyclic amino group include, for example, pyrrolidino, piperidino, morpholino, thiomorpholino, etc.

Examples of the optionally substituted phenyl group as (10) include, for example, a phenyl group which may be substituted with 1 to 4 (preferably 1 or 2) identical or different substituents selected from halogen atom, hydroxy group, nitro group, cyano group, a lower alkyl group optionally substituted with a halogen atom, a lower alkoxy group optionally substituted with a halogen atom, a lower acyl group and a mercapto group optionally substituted with a lower alkyl group, at any possible position.

Here, the halogen atom, the lower alkyl group optionally substituted with a halogen atom, the lower alkoxy group optionally substituted with a halogen atom, the lower acyl group and the mercapto group optionally substituted with a lower alkyl group, as the substituents for the phenyl group mentioned in the above (10), are similar to (1) halogen atom, (5) lower alkyl group optionally substituted with a halogen atom, (6) lower alkoxy group optionally substituted with a halogen atom, (7) lower acyl group and (8) mercapto group optionally substituted with a lower alkyl group, as the substituents for the benzene ring or nitrogen-containing 6-membered aromatic ring represented by the above ring A.

As the ring A, an optionally substituted benzene ring is preferable.

In the above formula (I), R¹ is an optionally substituted aromatic ring group.

Examples of the aromatic ring group represented by R¹ include, for example, a phenyl group, a pyridyl group (2-, 3- or 4-pyridyl), a thienyl group (2- or 3-thienyl), etc.

The aromatic ring group represented by R¹ may be substituted with 1 to 4 (preferably 1 or 2) identical or different substituents selected from, for example, a halogen atom, a hydroxy group, a nitro group, a cyano group, a lower alkyl group optionally substituted with a halogen atom, a lower alkoxy group optionally substituted with a halogen atom, a lower acyl group, a mercapto group optionally substituted with a lower alkyl group, an optionally substituted phenyl group, etc., at any possible position.

Here, the halogen atom, the lower alkyl group optionally substituted with a halogen atom, the lower alkoxy group optionally substituted with a halogen atom, the lower acyl group, the mercapto group optionally substituted with a lower alkyl group and an optionally substituted phenyl group, as the substituents for the aromatic ring group represented by R¹ are similar to (1) halogen atom, (5) lower alkyl group optionally substituted with a halogen atom, (6) lower alkoxy group optionally substituted with a halogen atom, (7) lower acyl group, (8) mercapto group optionally substituted with a lower alkyl group and (10) optionally substituted phenyl group, as the substituents for the benzene ring or nitrogen-containing 6-membered aromatic ring represented by the above ring A.

As R¹, an optionally substituted phenyl group or an optionally substituted thienyl group is preferable. Among others, a phenyl group or a thienyl group each of which may be substituted with a halogen atom or a lower alkyl group, and a phenyl group substituted with a lower alkyl group at the 2-position of the phenyl group (e.g. 2-methylphenyl, etc.) is more preferable.

Among the compound of the formula (I) [Compound (I)], a compound of the formula (Ia): 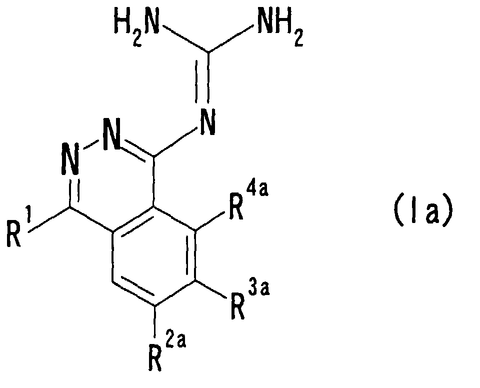 wherein R^{2a}, R^{3a} and R^{4a} are independently (1) a halogen atom, (2) a hydroxy group, (3) a nitro group, (4) a cyano group, (5) a lower alkyl group optionally substituted with a halogen atom, (6) a lower alkoxy group optionally substituted with a halogen atom, (7) a lower acyl group, (8) a mercapto group optionally substituted with a lower alkyl group, (9) an optionally substituted amino group or (10) an optionally substituted phenyl group, and the other symbol is as defined above, is preferable.

In the above formula (Ia), (1) halogen atom, (5) lower alkyl group optionally substituted with a halogen atom, (6) lower alkoxy group optionally substituted with a halogen atom, (7) lower acyl group, (8) mercapto group optionally substituted with a lower alkyl group, (9) optionally substituted amino group and (10) optionally substituted phenyl group represented by R^{2a}, R^{3a} and R^{4a} are similar to (1) halogen atom, (5) lower alkyl group optionally substituted with a halogen atom, (6) lower alkoxy group optionally substituted with a halogen atom, (7) lower acyl group, (8) mercapto group optionally substituted with a lower alkyl group and (10) optionally substituted phenyl group, as the substituents for the benzene ring or nitrogen-containing 6-membered aromatic ring represented by the above ring A.

As the groups R^{2a} and R^{3a}, a hydrogen atom is preferable.

As the group R^{4a}, a halogen atom or a lower alkyl group is preferable.

More preferable examples of Compound (I) include 8-iode-4-phenylphthalazin-1-ylguanidine or a salt thereof, 8-bromo-4-phenylphthalazin-1-ylguanidine or a salt thereof, 8-bromo-4-(2-methylphenyl)phthalazin-1-ylguanidine or a salt thereof, 8-iode-4-(2-methylphenyl)phthalazin-1-ylguanidine or a salt thereof, 8-bromo-4-(4-fluoro-2-methylphenyl)phthalazin-1-ylguanidine or a salt thereof, 8-bromo-4-(3-thienyl)phthalazin-1-ylguanidine or a salt thereof, 4-(4-fluorophenyl)-8-iodephthalazin-1-ylguanidine or a salt thereof, 8-methyl-4-(2-methylphenyl)phthalazin-1-ylguanidine or a salt thereof, etc., and in particular, 8-bromo-4-(2-methylphenyl)phthalazin-1-ylguanidine or a salt thereof, 8-methyl-4-(2-methylphenyl)phthalazin-1-ylguanidine or a salt thereof, etc. are preferable.

Examples of the salt of Compound (I) include a pharmaceutically acceptable salt, for example, an inorganic acid salt such as hydrochloride, hydrobromide, sulfate, nitrate, phosphate, etc., an organic acid salt such as acetate, tartarate, citrate, fumarate, maleate, toluenesulfonate, methanesulfonate, etc., a metal salt such as sodium salt, potassium salt, calcium salt, aluminum salt, etc., a salt with a base such as triethylamine salt, guanidine salt, ammonium salt, hydrazine salt, quinine salt, cinchonine salt, etc., etc.

Compound (I) may be used as a hydrate.

In the above formula (II), the ring C is an optionally substituted benzene ring.

The benzene ring represented by the ring C may be substituted with 1 to 4 identical or different substituents selected from, for example, (1) a halogen atom, (2) a hydroxy group, (3) a nitro group, (4) a cyano group, (5) a lower alkyl group optionally substituted with a halogen atom, (6) a lower alkoxy group optionally substituted with a halogen atom, (7) a lower acyl group, (8) a mercapto group optionally substituted with a lower alkyl group, (9) an optionally substituted amino group, (10) a carboxyl group optionally esterified or amidated, (11) a sulfino group (sulfinic acid group) or a sulfo group (sulfonic acid group), each of which may be esterified or amidated and (12) an optionally substituted phenyl group, at any possible position; and (13) adjoining two of these substituents may bind to each other to form a divalent hydrocarbon group which may contain a carbonyl group.

Examples of the halogen atom as (1) include, for example, chlorine, bromine, fluorine, iodine, etc.

Examples of the lower alkyl group optionally substituted with a halogen atom (for example, chlorine, bromine, fluorine, iodine, etc.) as (5) include, for example, a C₁₋₆ alkyl group (for example, methyl, ethyl, propyl, isopropyl, butyl, isobutyl, s-butyl, t-butyl, pentyl, hexyl, etc.), a halogeno C₁₋₆ alkyl group (for example, CF₃, CF₂CF₃, CH₂F, CHF₂), etc.

Examples of the lower alkoxy group optionally substituted with a halogen atom (for example, chlorine, bromine, fluorine, iodine, etc.) as (6) include, for example, a C₁₋₆ alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno C₁₋₆ alkoxy group (for example, CF₃O, CHF₂O, etc.), etc..

Examples of the lower acyl group as (7) include, for example, a lower acyl group derived from a carboxylic acid, a sulfinic acid, a sulfonic acid, etc.

Here, examples of the lower acyl group derived from a carboxylic acid include, for example, formyl, a lower (C₁₋₆) alkyl-carbonyl (C₁₋₆ alkanoyl) (for example, acetyl, propionyl, butyryl, isobutyryl, valeryl, pivaloyl, etc.), a C₃₋₆ cycloalkyl-carbonyl (for example, cyclopropylcarbonyl, cyclobutylcarbonyl, cyclopentylcarbonyl, cyclohexylcarbonyl, etc.), benzoyl, etc.

Examples of the lower acyl group derived from a sulfinic acid include, for example, a lower (C₁₋₆) alkylsulfinyl (for example, methylsulfinyl, ethylsulfinyl, propylsulfinyl, isopropylsulfinyl, butylsulfinyl, isobutylsulfinyl, s-butylsulfinyl, t-butylsulfinyl, pentylsulfinyl, hexylsulfinyl, etc.), a C₃₋₆ cycloalkylsulfinyl (for example, cyclopropylsulfinyl, cyclobutylsulfinyl, cyclopentylsulfinyl, cyclohexylsulfinyl, etc.), phenylsulfinyl, etc.

Examples of the lower acyl group derived from a sulfonic acid include, for example, a lower (C₁₋₆) alkylsulfonyl (for example, methylsulfonyl, ethylsulfonyl, propylsulfonyl, isopropylsulfonyl, butylsulfonyl, isobutylsulfonyl, t-butylsulfonyl, pentylsulfonyl, hexylsulfonyl, etc.), a C₃₋₆ cycloalkylsulfonyl (for example, cyclopropylsulfonyl, cyclobutylsulfonyl, cyclopentylsulfonyl, cyclohexylsulfonyl, etc.), phenylsulfonyl, etc.

These acyl groups may be substituted with, for example, 1 to 5 halogen atoms (for example, chlorine, bromine, fluorine, iodine, etc.) at any possible position, and examples of the substituted acyl group include CF₃CO, CF₃SO₂, etc.

Examples of the mercapto group optionally substituted with a lower alkyl group (for example, a C₁₋₆ alkyl such as methyl, ethyl, propyl, isopropyl, butyl, isobutyl, s-butyl, t-butyl, pentyl, hexyl, etc.) as (8) include, for example, a mercapto group, a C₁₋₆ alkylthiogroup (for example, methylthio, ethylthio, propylthio, isopropylthio, butylthio, isobutylthio, s-butylthio, t-butylthio, pentylthio, hexylthio, etc.), etc.

Examples of the optionally substituted amino group as (9) include, for example, amino groups which may be substituted with 1 or 2 identical or different substituents selected from lower (C₁₋₆) alkyl (for example, methyl, ethyl, propyl, isopropyl, butyl, isobutyl, s-butyl, t-butyl, pentyl, hexyl, etc.), lower (C₁₋₆) alkoxy (for example, methoxy, ethoxy, propoxy, isopropoxy, butoxy, isobutoxy, s-butoxy, t-butoxy, pentyloxy, hexyloxy, etc.), halogeno lower (C₁₋₆) alkyl (for example, CF₃, CF₃CF₂, CH₂F, CHF₂, etc.), lower (C₃₋₆) cycloalkyl (for example, cyclopropyl, cyclobutyl, cyclopentyl, cyclohexyl, etc.), hydroxy group, carbamoyl, phenyl, phenyl-lower (C₁₋₆) alkyl (for example, benzyl, phenethyl, 3-phenylpropyl, 4-phenylbutyl, etc.), formyl, lower (C₁₋₆) alkanoyl (for example, acetyl, propionyl, butyryl, isobutyryl, valeryl, pivaloyl, etc.), C₃₋₆ cycloalkyl-carbonyl (for example, cyclopropylcarbonyl, cyclobutylcarbonyl, cyclopentylcarbonyl, cyclohexylcarbonyl, etc.), benzoyl, phenyl-C₂₋₆ alkanoyl (for example, phenylacetyl, phenylpropionyl, etc.), lower (C₁₋₆) alkoxy-carbonyl (for example, methoxycarbonyl, ethoxycarbonyl, propoxycarbonyl, isopropoxycarbonyl, butoxycarbonyl, isobutoxycarbonyl, t-butoxycarbonyl, pentyloxycarbonyl, hexyloxycarbonyl, etc.), phenoxycarbonyl, phenyl-C₁₋₆ alkoxy-carbonyl (for example, benzyloxycarbonyl, phenylethoxycarbonyl, etc.), lower (C₁₋₆) alkylsulfinyl (for example, methylsulfinyl, ethylsulfinyl, propylsulfinyl, isopropylsulfinyl, butylsulfinyl, isobutylsulfinyl, s-butylsulfinyl, t-butylsulfinyl,pentylsulfinyl, hexylsulfinyl, etc.), C₃₋₆ cycloalkylsulfinyl (for example, cyclopropylsulfinyl, cyclobutylsulfinyl, cyclopentylsulfinyl, cyclohexylsulfinyl, etc.), phenylsulfinyl, lower (C₁₋₆) alkylsulfonyl (for example, methylsulfonyl, ethylsulfonyl, propylsulfonyl, isopropylsulfonyl, butylsulfonyl, isobutylsulfonyl, t-butylsulfonyl, s-butylsulfonyl, pentylsulfonyl, hexylsulfonyl, etc.), C₃₋₆ cycloalkylsulfonyl (for example, cyclopropylsulfonyl, cyclobutylsulfonyl, cyclopentylsulfonyl, cyclohexylsulfonyl, etc.), lower (C₁₋₆) alkoxysulfonyl (for example, methoxysulfonyl, ethoxysulfonyl, propoxysulfonyl, isopropoxysulfonyl, butoxysulfonyl, isobutoxysulfonyl, s-butoxysulfonyl, t-butoxysulfonyl, pentyloxysulfonyl, hexyloxysulfonyl, etc.), phenylsulfonyl, etc.

Also, two of the substituents may form a cyclic amino group in cooperation with a nitrogen atom, and examples of the cyclic amino group include, for example, pyrrolidino, piperidino, morpholino, thiomorpholino, etc.

Examples of the esterified carboxyl group in the carboxyl group optionally esterified or amidated as (10) include, for example, a lower (C₁₋₆) alkoxy-carbonyl group (for example, methoxycarbonyl, ethoxycarbonyl, propoxycarbonyl, isopropoxycarbonyl, butoxycarbonyl, s-butoxycarbonyl, t-butoxycarbonyl, pentyloxycarbonyl, isopentyloxycarbonyl, neopentyloxycarbonyl, hexyloxycarbonyl, etc.), a C₃₋₆ cycloalkoxy-carbonyl (for example, cyclopropoxycarbonyl, cyclobutyloxycarbonyl, cyclopentyloxycarbonyl, cyclohexyloxycarbonyl, etc.), a phenyl C₁₋₆ alkoxy-carbonyl (for example, benzyloxycarbonyl phenyloxycarbonyl, etc.), a nitroxy-C₁₋₆ alkoxy-carbonyl (for example, 2-nitroxyethoxycarbonyl, 3-nitroxypropoxycarbonyl, etc.), etc.

Examples of the amidated carboxyl group in the carboxyl group optionally esterified or amidated as (10) include a carbamoyl, a N-mono-lower (C₁₋₆) alkylcarbamoyl (for example, methylcarbamoyl, ethylcarbamoyl, propylcarbamoyl, isopropylcarbamoyl, butylcarbamoyl, isobutylcarbamoyl, s-butylcarbamoyl, t-butylcarbamoyl, pentylcarbamoyl, hexylcarbamoyl, etc.), a N,N-di-lower (C₁₋₆) alkylcarbamoyl (for example, N,N-dimethylcarbamoyl, N,N-diethylcarbamoyl, N,N-dipropylcarbamoyl, N,N-dibutylcarbamoyl, etc.), a C₃₋₆ cycloalkyl-carbamoyl (for example, cyclopropylcarbamoyl, cyclobutylcarbamoyl, cyclopentylcarbamoyl, cyclohexylcarbamoyl, etc.), a phenyl-C₁₋₆ alkyl-carbamoyl (for example, benzylcarbamoyl, phenethylcarbamoyl, etc.), a nitroxy-C₁₋₆ alkylaminocarbonyl (for example, 2-nitroxyethylcarbamoyl, 3-nitroxypropylcarbamoyl, etc.), a cyclic aminocarbonyl (for example, morpholinocarbonyl, piperidinocarbonyl, pyrrolidinocarbonyl, thiomorpholinocarbonyl, etc.), an anilinocarbonyl, etc.

Examples of the esterified sulfino group in the sulfino group or a sulfo group, each of which may be esterified or amidated as (11) include, for example, a lower (C₁₋₆) alkoxysulfinyl (for example, methoxysulfinyl, ethoxysulfinyl, propoxysulfinyl, isopropoxysulfinyl, butoxysulfinyl, isobutoxysulfinyl, s-butoxysulfinyl, t-butoxysulfinyl, pentyloxysulfinyl, hexyloxysulfinyl, etc.), a C₃₋₆ cycloalkylsulfinyl (for example, cyclopropoxysulfinyl, cyclobutyloxysulfinyl, cyclopentyloxysulfinyl, cyclohexyloxysulfinyl, etc.), a phenyl C₁₋₆ alkoxysulfinyl (for example, benzyloxysulfinyl, phenethyloxysulfinyl, etc.), etc.

Examples of the amidated sulfino group in the sulfino group or a sulfo group, each of which may be esterified or amidated as (11) include, for example, a sulfinamoyl, a lower (C₁₋₆) alkyl-aminosulfinyl (for example, methylaminosulfinyl, ethylaminosulfinyl, propylaminosulfinyl, isopropylaminosulfinyl, butylaminosulfinyl, isobutylaminosulfinyl, s-butylaminosulfinyl, t-butylaminosulfinyl, pentylaminosulfinyl, hexylaminosulfinyl, etc.), a C₃₋₆ cycloalkylaminosulfinyl (for example, cyclopropylaminosulfinyl, cyclobutylaminosulfinyl, cyclopentylaminosulfinyl, cyclohexylaminosulfinyl, etc.), a phenyl-C₁₋₄ alkylamino-sulfinyl (for example, benzylaminosulfinyl, phenethylaminosulfinyl, etc.), cyclic aminosulfinyl (for example, morpholinosulfinyl, piperidinosulfinyl, pyrrolidinosulfinyl, thiomorpholinosulfinyl, etc.), a nitroxy-C₁₋₆ alkylamino-sulfinyl (for example, 2-nitroxyethylaminosulfinyl, 3-nitroxypropylaminosulfinyl, etc.), an anilinosulfinyl, etc.

Examples of the esterified sulfo group in the sulfino group or a sulfo group, each of which may be esterified or amidated as (11) include, for example, a lower (C₁₋₆) alkoxysulfonyl (for example, methoxysulfonyl, ethoxysulfonyl, propoxysulfonyl, isopropoxysulfonyl, butoxysulfonyl, isobutoxysulfonyl, s-butoxysulfonyl, t-butoxysulfonyl, pentyloxysulfonyl, hexyloxysulfonyl, etc.), a C₃₋₆ cycloalkylsulfonyl (for example, cyclopropoxysulfonyl, cyclobutyloxysulfonyl, cyclopentyloxysulfonyl, cyclohexyloxysulfonyl, etc.), a phenyl C₁₋₆ alkoxysulfonyl (for example, benzyloxysulfonyl, phenethyloxysulfonyl, etc.), etc.

Examples of the amidated sulfo group in the sulfino group or a sulfo group, each of which may be esterified or amidated as (11) include, for example, a sulfamoyl, a lower (C₁₋₆) alkylaminosulfonyl (for example, methylaminosulfonyl, ethylaminosulfonyl, propylaminosulfonyl, isopropylaminosulfonyl. butylaminosulfonyl, isobutylaminosulfonyl, s-butylaminosulfonyl, t-butylaminosulfonyl, pentylaminosulfonyl, hexylaminosulfonyl, etc.), a C₃₋₆ cycloalkylaminosulfonyl (for example, cyclopropylaminosulfonyl, cyclobutylaminosulfonyl, cyclopentylaminosulfonyl, cyclohexylaminosulfonyl, etc.), a phenyl C₁₋₆ alkylaminosulfonyl (for example, benzylaminosulfonyl, phenethylaminosulfonyl, etc.), a cyclic aminosulfonyl (for example, morpholinosulfonyl, piperidinosulfonyl, pyrrolidinosulfonyl, thiomorpholinosulfonyl, etc.), a nitroxy-C₁₋₆ alkylamino-sulfonyl (for example, 2-nitroxyethylaminosulfonyl, 3-nitroxypropylaminosulfonyl, etc.), an anilinosulfonyl, etc.

Examples of the optionally substituted phenyl group as (12) include, for example, a phenyl group which may be substituted with 1 to 3 identical or different substituents selected from halogen atom (for example, chlorine, bromine, fluorine, iodine, etc.), hydroxy group, nitro group, cyano group, lower (C₁₋₆) alkyl group (for example, methyl, ethyl, propyl, isopropyl, butyl, isobutyl, s-butyl, t-butyl, pentyl, hexyl, etc.), halogeno lower (C₁₋₆) alkyl group (for example, CF₃, CF₂CF₃, CH₂F, CHF₂, etc.), lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), halogeno lower (C₁₋₆) alkoxy group (for example, CF₃O, CHF₂O, etc.), etc., at any possible position.

Examples of the divalent hydrocarbon group which may contain a carbonyl group as (13) include, for example, a group of the formula:
-C(Ra)=C(Rb)-C(Rc)=C(Rd)-,
-(CReRf)a- (a is 3 or 4),
-(CRgRh)b-CO- (b is 2 or 3), etc.

Here, Ra, Rb, Rc, Rd, Re, Rf, Rg and Rh are independently hydrogen atom, lower (C₁₋₆) alkyl group (for example, methyl, ethyl, propyl, isopropyl, butyl, isobutyl, s-butyl, t-butyl, pentyl, hexyl, etc.), halogen atom (for example, chlorine, bromine, fluorine, iodine, etc.), lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), halogeno lower (C₁₋₆) alkyl group (for example, CF₃, CF₂CF₃, CH₂F, CHF₂, etc.), lower (C₁₋₆) alkoxy-carbonyl group (for example, methoxycarbonyl, ethoxycarbonyl, propoxycarbonyl, isopropoxycarbonyl, butoxycarbonyl, s-butoxycarbonyl, t-butoxycarbonyl, pentyloxycarbonyl, isopentyloxycarbonyl, neopentyloxycarbonyl, hexyloxycarbonyl, etc.), cyano group and nitro group.

In the above formula (II), the ring D is an optionally substituted nitrogen-containing 6-membered aromatic ring group which has a nitrogen atom next to the position of a chemical bond (at the α-position)

Examples of the nitrogen-containing 6-membered aromatic ring represented by the ring D include, for example, a pyridine ring (2-pyridyl), a pyrimidine ring (2- or 4-pyrimidyl), a pyrazine ring (2-pyrazyl), a pyridazine ring (3-pyridazyl), etc.

The nitrogen-containing 6-membered aromatic ring represented by the ring D may be substituted with 1 to 4 (preferably 1 or 2) identical or different substituents selected from, for example, a halogen atom, a hydroxy group, a nitro group, a cyano group, a lower alkyl group optionally substituted with a halogen atom, a lower alkoxy group optionally substituted with a halogen atom, a lower acyl group, an optionally substituted amino group, a carboxyl group optionally esterified or amidated, an optionally substituted phenyl group, etc., at any possible position.

Here, the halogen atom, the lower alkyl group optionally substituted with a halogen atom, the lower alkoxy group optionally substituted with a halogen atom, the lower acyl group, the optionally substituted amino group, the carboxyl group optionally esterified or amidated and the optionally substituted phenyl group, as the substituents for the nitrogen-containing 6-membered aromatic ring represented by the ring D are similar to (1) halogen atom, (5) lower alkyl group optionally substituted with a halogen atom, (6) lower alkoxy group optionally substituted with a halogen atom, (7) lower acyl group, (9) optionally substituted amino group, (10) carboxyl group optionally esterified or amidated and (12)optionally substituted phenyl group, as the substituents for the benzene ring represented by the above ring C.

In the above formula (II), as the ring D, a nitrogen-containing 6-membered aromatic ring group which has a nitrogen atom next to the position of a chemical bond (at the α-position) and which may be substituted with 1 to 4 identical or different substituents selected from a halogen atom, a hydroxy group, a nitro group, a cyano group, a lower alkyl group optionally substituted with a halogen atom, a lower alkoxy group optionally substituted with a halogen atom, a lower acyl group, an optionally substituted amino group, a mercapto group optionally substituted with a lower alkyl group, a carboxyl group optionally esterified or amidated and an optionally substituted phenyl group, at any possible position.

Examples of the salt of a compound of the formula (II) [Compound (II)] include a pharmaceutically acceptable salt, for example, an inorganic acid salt such as hydrochloride, hydrobromide, sulfate, nitrate, phosphate, etc., an organic acid salt such as acetate, tartarate, citrate, fumarate, maleate, toluenesulfonate, methanesulfonate, etc., a metal salt such as sodium salt, potassium salt, calcium salt, aluminum salt, etc., a salt with a base such as triethylamine salt, guanidine salt, ammonium salt, hydrazine salt, quinine salt, cinchonine salt, etc., etc.

Compound (II) may be used as a hydrate.

Compound (II) has geometrical isomerism at the position of an oxime structure based on the configuration of a pyridyl group and a guanidyl group, and exists as E-isomer, Z-isomer or a mixture thereof. In Compound (II), individual isomers and a mixture thereof are included.

In the above formula (III), the ring E is an optionally substituted 5- to 6-membered aromatic heterocyclic ring.

Examples of the aromatic heterocyclic ring in the "optionally substituted 5- to 6-membered aromatic heterocyclic ring" represented by E include, for example, an aromatic heterocyclic ring containing at least one hetero-atom (preferably 1 to 3 hetero-atoms, more preferably 1 to 2 hetero-atoms) consisting of 1 to 3 kinds (preferably 1 to 2 kinds) of hetero-atoms selected from an oxygen atom, a sulfur atom, a nitrogen atom, etc., as an atom constituting ring structure (ring atom).

Examples of said "aromatic heterocyclic ring" include, for example, a 5- to 6-membered aromatic heterocyclic ring, etc. such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazane, 1,2,3-thiadiazole, 1,2,4-thiadiazole, 1,3,4-thiadiazole, 1,2,3-triazole, 1,2,4-triazole, pyridine, pyridazine, pyrimidine, pyrazine, triazine, etc.), etc.

Among others, a 5 - to 6-membered aromatic heterocyclic ring containing 1 to 3 hetero-atoms (preferably 1 to 2 hetero-atoms) selected from an oxygen atom, a sulfur atom and a nitrogen atom is preferable, and preferred examples of the ring E include a pyridine ring, a pyridazine ring, a pyrrole ring, a pyrazole ring, a furan ring, a thiophene ring, an isoxazole ring, a pyrimidine ring (preferably, a 5- to 6-membered nitrogen-containing aromatic heterocyclic ring which contains 1-2 nitrogen atoms, etc. such as a pyridine ring, a pyridazine ring, a pyrrole ring, a pyrazole ring, etc.).

In the above formula (III), the ring F is an optionally substituted 5- to 6-membered aromatic homo- or hetero-cyclic ring.

Examples of the "optionally substituted 5- to 6-membered aromatic homocyclic ring" represented by F include an optionally substituted benzene ring, etc.

Examples of the aromatic heterocyclic ring in the "optionally substituted 5- to 6-membered aromatic heterocyclic ring" represented by F include, for example, an aromatic heterocyclic ring containing at least one hetero-atom (preferably 1 to 3 hetero-atoms, more preferably 1 to 2 hetero-atoms) consisting of 1 to 3 kinds (preferably 1 to 2 kinds) of hetero-atoms selected from an oxygen atom, a sulfur atom, a nitrogen atom, etc., as an atom constituting ring structure (ring atom).

Examples of said "aromatic heterocyclic ring" include, for example, a 5- to 6-membered aromatic heterocyclic ring, etc. such as furan, thiophene, pyrrole, oxazole, isoxazole, thiazole, isothiazole, imidazole, pyrazole, 1,2,3-oxadiazole, 1,2,4-oxadiazole, 1,3,4-oxadiazole, furazane, 1,2,3-thiadiazole, 1,2,4-thiadiazole, 1,3,4-thiadiazole, 1,2,3-triazole, 1,2,4-triazole, pyridine, pyridazine, pyrimidine, pyrazine, triazine, etc.), etc.

Among others, a 5- to 6-membered aromatic heterocyclic ring containing 1 to 3 hetero-atoms (preferably 1 to 2 hetero-atoms) selected from an oxygen atom, a sulfur atom and a nitrogen atom is preferable, and pyridine, pyridazine, pyrrole, pyrazole, furan, thiophene, isoxazole, pyrimidine, etc. (preferably a 5- to 6-membered aromatic heterocyclic ring containing 1 to 2 nitrogen-atoms such as pyridine, pyridazine, pyrrole, pyrazole, etc.; more preferably a 5- to 6-membered aromatic heterocyclic ring containing 1 to 2 nitrogen-atoms such as pyridine, pyridazine, pyrazole, etc.) are more preferable

Preferred examples of the ring F include a 5- to 6-membered aromatic homo- or hetero-cyclic ring which may contain one hetero-atom selected from an oxygen atom, a sulfur atom and a nitrogen atom, etc. such as a benzene ring, a pyrrole ring, a furan ring, a thiophene ring, a pyridine ring (preferably a benzene ring, a furan ring, a thiophene ring, etc.), etc.

Both of the ring E and the ring F may be substituted with 1 to 4 (preferably 1 to 2) identical or different substituents selected from, for example, (1) a halogen atom, (2) a hydroxy group, (3) a nitro group, (4) a cyano group, (5) an optionally substituted lower alkyl group, (6) an optionally substituted lower alkenyl group, (7) an optionally substituted lower alkynyl group, (8) an optionally substituted lower aralkyl group, (9) an optionally substituted lower alkoxy group, (10) an optionally substituted mercapto group, (11) an optionally substituted amino group, (12) a carboxyl group optionally esterified or amidated, (13) an optionally substituted sulfonyl group and (14) an optionally substituted acyl group, at any possible position; and (15) adjoining two of these substituents may bind to each other to form a divalent hydrocarbon group; and a nitrogen atom of the ring E and the ring F may be oxidized.

Examples of the halogen atom as (1) include, for example, chlorine, bromine, fluorine, iodine, etc.

Examples of the lower alkyl group in the optionally substituted lower alkyl group as (5) include, for example, a C₁₋₆ alkyl group (for example, methyl, ethyl, propyl, isopropyl, butyl, isobutyl, s-butyl, t-butyl, pentyl, hexyl, etc.), etc.

Said lower alkyl group may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the lower alkenyl group in the optionally substituted lower alkenyl group as (6) include, for example, a C₂₋₆ alkenyl group, etc. such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc.

Said lower alkenyl group may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the lower alkynyl group in the optionally substituted lower alkynyl group as (7) include, for example, a C₂₋₆ alkenyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc.

Said lower alkynyl group may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the lower aralkyl group in the optionally substituted lower aralkyl group as (8) include, for example, a C₇₋₁₀ aralkyl group (preferably, phenyl-C₁₋₆ alkyl group, etc.), etc. such as benzyl, phenethyl, etc.

Said lower aralkyl group may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkyl group (for example, methyl, ethyl, propyl, isopropyl, butyl, isobutyl, s-butyl, t-butyl, pentyl, hexyl, etc.), a halogeno lower (C₁₋₆) alkyl group (for example, CF₃, CF₂CF₃, CH₂F, CHF₂, etc.), a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the lower alkoxy group in the optionally substituted lower alkoxy group as (9) include, for example, a C₁₋₆ alkoxy group (for example, methoxy, ethoxy, propoxy, isopropoxy, butoxy, s-butoxy, t-butoxy, pentyloxy, isopentyloxy, neopentyloxy, hexyloxy, etc.), etc.

Said lower alkoxy group may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the optionally substituted mercapto group as (10) include, for example, an optionally substituted C₁₋₆ alkylthio group (for example, methylthio, ethylthio, propylthio, isopropylthio, butylthio, s-butylthio, t-butylthio, pentylthio, isopentylthio, neopentylthio, hexylthio, etc.), etc.

Said C₁₋₆ alkylthio group may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the optionally substituted amino group as (11) include, for example, amino groups which may be substituted with 1 or 2 identical or different substituents selected from lower (C₁₋₆) alkyl (for example, methyl, ethyl, propyl, isopropyl, butyl, isobutyl, s-butyl, t-butyl, pentyl, hexyl, etc.), lower (C₁₋₆) alkoxy (for example, methoxy, ethoxy, propoxy, isopropoxy, butoxy, isobutoxy, s-butoxy, t-butoxy, pentyloxy, hexyloxy, etc.), halogeno lower (C₁₋₆) alkyl (for example, CF₃, CF₃CF₂, CH₂F, CHF₂, etc.), lower (C₃₋₆)cycloalkyl (for example, cyclopropyl, cyclobutyl, cyclopentyl, cyclohexyl, etc.), hydroxy group, carbamoyl, phenyl, phenyl-lower (C₁₋₆) alkyl (for example, benzyl, phenethyl, 3-phenylpropyl, 4-phenylbutyl, etc.), formyl, lower (C₁₋₆) alkanoyl (for example, acetyl, propionyl, butyryl, isobutyryl, valeryl, pivaloyl, etc.), C₃₋₆ cycloalkyl-carbonyl (for example, cyclopropylcarbonyl, cyclobutylcarbonyl, cyclopentylcarbonyl, cyclohexylcarbonyl, etc.), benzoyl, phenyl-C₂₋₆ alkanoyl (for example, phenylacetyl, phenylpropionyl, etc.), lower (C₁₋₆) alkoxy-carbonyl (for example, methoxycarbonyl, ethoxycarbonyl, propoxycarbonyl, isopropoxycarbonyl, butoxycarbonyl, isobutoxycarbonyl, t-butoxycarbonyl, pentyloxycarbonyl, hexyloxycarbonyl, etc.), phenoxycarbonyl, phenyl-C₁₋₆ alkoxy-carbonyl (for example, benzyloxycarbonyl, phenylethoxycarbonyl, etc.), lower (C₁₋₆) alkylsulfinyl (for example, methylsulfinyl, ethylsulfinyl, propylsulfinyl, isopropylsulfinyl, butylsulfinyl, isobutylsulfinyl, s-butylsulfinyl, t-butylsulfinyl,pentylsulfinyl, hexylsulfinyl, etc.), C₃₋₆ cycloalkylsulfinyl (for example, cyclopropylsulfinyl, cyclobutylsulfinyl, cyclopentylsulfinyl, cyclohexylsulfinyl, etc.), phenylsulfinyl, lower (C₁₋₆) alkylsulfonyl (for example, methylsulfonyl, ethylsulfonyl, propylsulfonyl, isopropylsulfonyl, butylsulfonyl, isobutylsulfonyl, t-butylsulfonyl, s-butylsulfonyl, pentylsulfonyl, hexylsulfonyl, etc-), C₃₋₆ cycloalkylsulfonyl (for example, cyclopropylsulfonyl, cyclobutylsulfonyl, cyclopentylsulfonyl, cyclohexylsulfonyl, etc.), lower (C₁₋₆) alkoxysulfonyl (for example, methoxysulfonyl, ethoxysulfonyl, propoxysulfonyl, isopropoxysulfonyl, butoxysulfonyl, isobutoxysulfonyl, s-butoxysulfonyl, t-butoxysulfonyl, pentyloxysulfonyl, hexyloxysulfonyl, etc.), phenylsulfonyl, etc.

Also, two of the substituents may form a cyclic amino group in cooperation with a nitrogen atom, and examples of the cyclic amino group include, for example, pyrrolidino, piperidino, morpholino, thiomorpholino, etc.

Each of the optionally substituted amino groups as exemplified above may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkyl group (for example, methyl, ethyl, propyl, isopropyl, butyl, isobutyl, s-butyl, t-butyl, pentyl, hexyl, etc.), a halogeno lower (C₁₋₆) alkyl group (for example, CF₃, CF₂CF₃, CH₂F, CHF₂, etc.), a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the esterified carboxyl group in the carboxyl group optionally esterified or amidated as (12) include, for example, a lower (C₁₋₆) alkoxy-carbonyl group (for example, methoxycarbonyl, ethoxycarbonyl, propoxycarbonyl, isopropoxycarbonyl, butoxycarbonyl, s-butoxycarbonyl, t-butoxycarbonyl, pentyloxycarbonyl, isopentyloxycarbonyl, neopentyloxycarbonyl, hexyloxycarbonyl, etc.), a C₃₋₆ cycloalkoxy-carbonyl (for example, cyclopropoxycarbonyl, cyclobutyloxycarbonyl, cyclopentyloxycarbonyl, cyclohexyloxycarbonyl, etc.), a phenyl-C₁₋₆ alkoxy-carbonyl (for example, benzyloxycarbonyl, phenyloxycarbonyl, etc.), a nitroxy-C₁₋₆ alkoxy-carbonyl (for example, 2-nitroxyethoxycarbonyl, 3-nitroxypropoxycarbonyl, etc.), etc.

Examples of the amidated carboxyl group in the carboxyl group optionally esterified or amidated as (12) include a carbamoyl, a N-mono-lower (C₁₋₆) alkylcarbamoyl (for example, methylcarbamoyl, ethylcarbamoyl, propylcarbamoyl, isopropylcarbamoyl, butylcarbamoyl, isobutylcarbamoyl, s-butylcarbamoyl, t-butylcarbamoyl, pentylcarbamoyl, hexylcarbamoyl, etc.), a N,N-di-lower (C₁₋₆) alkylcarbamoyl (for example, N,N-dimethylcarbamoyl, N,N-diethylcarbamoyl, N,N-dipropylcarbamoyl, N,N-dibutylcarbamoyl, etc.), a C₃₋₆ cycloalkyl-carbamoyl (for example, cyclopropylcarbamoyl, cyclobutylcarbamoyl, cyclopentylcarbamoyl, cyclohexylcarbamoyl, etc.), a phenyl-C₁₋₆ alkyl-carbamoyl (for example, benzylcarbamoyl, phenethylcarbamoyl, etc.), a nitroxy-C₁₋₆ alkylaminocarbonyl (for example, 2-nitroxyethylcarbamoyl, 3-nitroxypropylcarbamoyl, etc.), a cyclic aminocarbonyl (for example, morpholinocarbonyl, piperidinocarbonyl, pyrrolidinocarbonyl, thiomorpholinocarbonyl, etc.), an anilinocarbonyl, etc.

Each of the "carboxyl group optionally esterified or amidated" as exemplified above may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkyl group (for example, methyl, ethyl, propyl, isopropyl, butyl, isobutyl, s-butyl, t-butyl, pentyl, hexyl, etc.), a halogeno lower (C₁₋₆) alkyl group (for example, CF₃, CF₂CF₃, CH₂F, CHF₂, etc.), a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc. at any possible position.

Examples of the optionally substituted sulfonyl group as (13) include, for example, a lower (C₁₋₆) alkylsulfonyl (for example, methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, hexylsulfonyl, etc.), a C₃₋₆ cycloalkylsulfonyl (for example, cyclopropylsulfonyl, cyclobutylsulfonyl, cyclopentylsulfonyl, cyclohexylsulfonyl, etc.), a phenyl-C₁₋₆ alkylsulfonyl (for example, benzylsulfonyl, phenethylsulfonyl, etc.), a lower (C₁₋₆) alkoxysulfonyl (for example, methoxysulfonyl, ethoxysulfonyl, propoxysulfonyl, isopropoxysulfonyl, butoxysulfonyl, isobutoxysulfonyl, s-butoxysulfonyl, t-butoxysulfonyl, pentyloxysulfonyl, hexyloxysulfonyl, etc.), a C₃₋₆ cycloalkyloxysulfonyl (for example, cyclopropoxysulfonyl, cyclobutyloxysulfonyl, cyclopentyloxysulfonyl, cyclohexyloxysulfonyl, etc.), a phenyl-C₁₋₆alkoxysulfonyl (for example, benzyloxysulfonyl, phenethyloxysulfonyl, etc.), a sulfamoyl, a lower (C₁₋₆) alkylaminosulfonyl (for example, methylaminosulfonyl, ethylaminosulfonyl, propylaminosulfonyl, isopropylaminosulfonyl, butylaminosulfonyl, isobutylaminosulfonyl, s-butylaminosulfonyl, t-butylaminosulfonyl, pentylaminosulfonyl, hexylaminosulfonyl, etc.), a C₃₋₆ cycloalkylaminosulfonyl (for example, cyclopropylaminosulfonyl, cyclobutylaminosulfonyl, cyclopentylaminosulfonyl, cyclohexylaminosulfonyl, etc.), a phenyl-C₁₋₆ alkylaminosulfonyl (for example, benzylaminosulfonyl, phenethylaminosulfonyl, etc.), a cyclic aminosulfonyl (for example, morpholinosulfonyl, piperidinosulfonyl, pyrrolidinosulfonyl, thiomorpholinosulfonyl, etc.), a nitroxy-C₁₋₆ alkylamino-sulfonyl (for example, 2-nitroxyethylaminosulfonyl, 3-nitroxypropylaminosulfonyl, etc.), an anilinosulfonyl, etc.

Each of the "optionally substituted sulfonyl group" as exemplified above may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkyl group (for example, methyl, ethyl, propyl, isopropyl, butyl, isobutyl, s-butyl, t-butyl, pentyl, hexyl, etc.), a halogeno lower (C₁₋₆) alkyl group (for example, CF₃, CF₂CF₃, CH₂F, CHF₂, etc.), a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the lower acyl group as (14) include, for example, a lower acyl group derived from a carboxylic acid, a sulfinic acid, a sulfonic acid, etc.

Here, examples of the lower acyl group derived from a carboxylic acid include, for example, formyl, a lower (C₁₋₆) alkyl-carbonyl (C₁₋₆ alkanoyl) (for example, acetyl, propionyl, butyryl, isobutyryl, valeryl, pivaloyl, etc.), a C₃₋₆ cycloalkyl-carbonyl (for example, cyclopropylcarbonyl, cyclobutylcarbonyl, cyclopentylcarbonyl, cyclohexylcarbonyl, etc.), benzoyl, etc.

Examples of the lower acyl group derived from a sulfinic acid include, for example, a lower (C₁₋₆) alkylsulfinyl (for example, methylsulfinyl, ethylsulfinyl, propylsulfinyl, isopropylsulfinyl, butylsulfinyl, isobutylsulfinyl, s-butylsulfinyl, t-butylsulfinyl, pentylsulfinyl, hexylsulfinyl, etc.), a C₃₋₆ cycloalkylsulfinyl (for example, cyclopropylsulfinyl, cyclobutylsulfinyl, cyclopentylsulfinyl, cyclohexylsulfinyl, etc.), phenylsulfinyl, etc.

Examples of the lower acyl group derived from a sulfonic acid include, for example, a lower (C₁₋₆) alkylsulfonyl (for example, methylsulfonyl, ethylsulfonyl, propylsulfonyl, isopropylsulfonyl, butylsulfonyl, isobutylsulfonyl, s-butylsulfonyl, t-butylsulfonyl, pentylsulfonyl, hexylsulfonyl, etc.), a C₃₋₆ cycloalkylsulfonyl (for example, cyclopropylsulfonyl, cyclobutylsulfonyl, cyclopentylsulfonyl, cyclohexylsulfonyl, etc.), phenylsulfonyl, etc.

Each of the "lower acyl group" as exemplified above may be substituted with 1 to 3 identical or different substituents selected from, for example, a halogen atom (for example, chlorine, bromine, fluorine, iodine, etc.), a hydroxy group, a nitro group, a cyano group, a lower (C₁₋₆) alkyl group (for example, methyl, ethyl, propyl, isopropyl, butyl, isobutyl, s-butyl, t-butyl, pentyl, hexyl, etc.), a halogeno lower (C₁₋₆) alkyl group (for example, CF₃, CF₂CF₃, CH₂F, CHF₂, etc.), a lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), a halogeno lower (C₁₋₆) alkoxy group (for example, CF₃O, CHF₂O, etc.), etc., at any possible position.

Examples of the divalent hydrocarbon group which may contain a carbonyl group as (15) include, for example, a group of the formula:
-CH=CH-CH=CH-,
-CH=CH-CH₂-CH₂-,
-CH₂-CH=CH₂-CH₂-,
-CH=CH-CH₂,
-(CH₂)a- (a is 3 or 4), etc.

Here, the above-mentioned divalent hydrocarbon group forms a 5- to 6-membered ring with two atoms constituting the ring E, and said 5- to 6-membered ring may be substituted with 1 to 3 identical or different substituents selected from, for example, lower (C₁₋₆) alkyl group (for example, methyl, ethyl, propyl, isopropyl, butyl, isobutyl, s-butyl, t-butyl, pentyl, hexyl, etc.), halogen atom (for example, chlorine, bromine, fluorine, iodine, etc.), lower (C₁₋₆) alkoxy group (for example, methoxy, ethoxy, propoxy, isopropoxy, butoxy, s-butoxy, t-butoxy, pentyloxy, isopentyloxy, neopentyloxy, hexyloxy, etc.), halogeno lower (C₁₋₆) alkyl group (for example, CF₃, CF₂CF₃, CH₂F, CHF₂, etc.), halogeno lower (C₁₋₆) alkoxy group (for example, CF₃O, CF₂CF₃O, CH₂FO, CHF₂O, etc.), lower (C₁₋₆) alkoxy-carbonyl group (for example, methoxycarbonyl, ethoxycarbonyl, propoxycarbonyl, isopropoxycarbonyl, butoxycarbonyl, s-butoxycarbonyl, t-butoxycarbonyl, pentyloxycarbonyl, isopentyloxycarbonyl, neopentyloxycarbonyl, hexyloxycarbonyl, etc.), cyano group, nitro group, hydroxy group, etc., at any possible position.

As the substituent for the ring E, a lower (C₁₋₆) alkyl group (preferably methyl) optionally halogenated, a lower (C₁₋₆) alkoxy group (preferably methoxy) optionally halogenated, etc. are preferable.

As the ring E, a group of the formula: 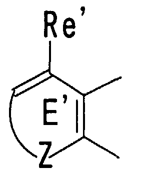 wherein the ring E' is an optionally substituted 5- to 6-membered aromatic heterocyclic ring (preferably, pyridine, pyrazole, pyrrole, furan, more preferably, pyridine, pyrazole) which may have an optional substituent in addition to the group Re', Z is an oxygen atom, a sulfur atom or a nitrogen atom, and Re' is the above-mentioned substituent for the ring E (preferably, lower (C₁₋₆) alkyl group optionally halogenated, lower (C₁₋₆) alkoxy group optionally halogenated, etc.) is preferable.

As the substituent for the ring F, halogen atom (preferably chlorine), lower (C₁₋₆) alkyl group (preferably methyl) optionally halogenated, hydroxy group, lower (C₁₋₆) alkoxy group (preferably methoxy) optionally halogenated, etc. are preferable.

As the ring F, a group of the formula: 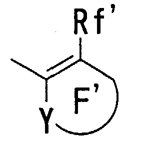 wherein the ring F' is an optionally substituted 5- to 6-membered aromatic homo- or hetero-cyclic ring (preferably, benzene, thiophene) in addition to the group Rf', Y is a carbon atom, an oxygen atom, a sulfur atom or a nitrogen atom and Rf' is hydrogen atom or the above-mentioned substituent for the ring F (preferably halogen atom, lower (C₁₋₆) alkyl group optionally halogenated, hydroxy group, lower (C₁₋₆) alkoxy group optionally halogenated, etc.) is preferable.

In the above formula (III), R² is a hydrogen atom, a hydroxy group or a lower alkyl group (for example, lower (C₁₋₆) alkyl group (for example, methyl, ethyl, propyl, isopropyl, butyl, isobutyl, s-butyl, t-butyl, pentyl, hexyl, etc.), preferably methyl). As the group R², hydrogen atom, hydroxy group and methyl group are preferable, hydrogen atom and hydroxy group are more preferable, and in particular, hydrogen atom is preferable.

In the above formula (III), n is 0 or 1 (preferably, 1).

As the compound of the formula (III) [Compound (III)],
(S)-(-)-7-(2,5-dichlorothiophen-3-yl)-5-guanidinoimino-4-methyl-5,6,7,8-tetrahydroquinoline;
(±)-7-(2,5-dichlorothiophen-3-yl)-5-guanidinoimino-4-methyl-5,6,7,8-tetrahydroquinoline;
(S)-(-)-7-(2-chlorophenyl)-5-guanidinoimino-4-methyl-5,6,7,8-tetrahydroquinoline;
(±)-7-(2-chlorophenyl)-5-guanidinoimino-4-methyl-5,6,7,8-tetrahydroquinoline;
(±)-7-(2,5-dichlorophenyl)-5-guanidinoimino-4-methyl-5,6,7,8-tetrahydrocinnoline;
(±)-7-(5-chloro-2-methylphenyl)-5-guanidinolmino-4-methyl-5,6,7,8-tetrahydroquinoline;
(±)-7-(5-fluoro-2-methylphenyl)-5-guanidinoimino-4-methyl-5,6,7,8-tetrahydroquinoline;
(±)-7-(5-fluoro-2-methylphenyl)-5-guanidinoimino-4-methyl-5,6,7,8-tetrahydrocinnoline
(±)-7-(2-chloro-5-fluorophenyl)-5-guanidinoimino-4-methyl-5,6,7,8-tetrahydroquinoline;
(±)-7-(5-chloro-2-fluorophenyl)-5-guanidinoimino-4-methyl-5,6,7,8-tetrahydroquinoline;
(±)-7-(5-chloro-2-fluorophenyl)-5-guanidinoimino-4-methyl-5,6,7,8-tetrahydrocinnoline;
(±)-7-(2-bromophenyl)-5-guanidinoimino-4-methyl-5,6,7,8-tetrahydroquinoline;
7-(3,5-dichlorothiophen-2-yl)-5-guanidinoimino-4-methyl-5,6,7,8-tetrahydroquinoline;
6-(2,5-dichlorothiophen-3-yl)-4-guanidinoimino-3-methyl-4,5,6,7-tetrahydroindazol;
or a salt thereof are preferable.

Examples of the salt of a compound of the formula (III) [Compound (III)] include a pharmaceutically acceptable salt, for example, an inorganic acid salt such as hydrochloride, hydrobromide, sulfate, nitrate, phosphate, etc., an organic acid salt such as acetate, tartarate, citrate, fumarate, maleate, toluenesulfonate, methanesulfonate, etc., a salt with an amino acid such as an aspartic acid, a glutamic acid, an arginine, a lysine, ornithine, etc., a metal salt such as sodium salt, potassium salt, calcium salt, aluminum salt, etc., a salt with a base such as triethylamine salt, guanidine salt, ammonium salt, hydrazine salt, quinine salt, cinchonine salt, etc., etc.

Compound (III) may be used as a hydrate.

When Compound (III) exists as configuration isomer, diastereomer, conformer, etc., it is possible to isolate individual isomers with per se known separation and purification method, if desired.

Compound (III) has geometrical isomerism at the position of a hydrazone structure based on cofiguration of a fused heterocyclic ring including the ring E, and exists as E-isomer, Z-isomer or a mixture thereof.

Further, when R² is a hydroxy group or a lower alkyl group, Compound (III) has geometrical isomerism based on double bond of a guanidino group, and exists as E-isomer, Z-isomer or a mixture thereof.

In Compound (III), the following individual isomers and a mixture thereof are included.

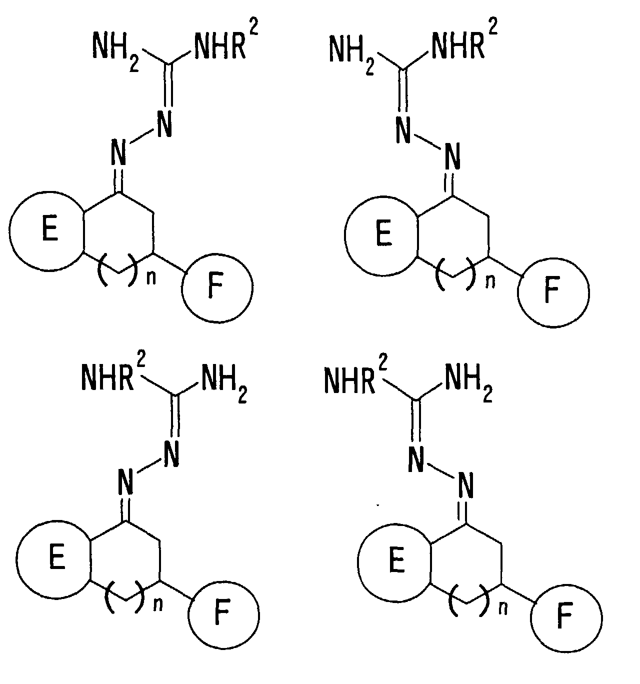

Also, Compound (III) has an optical isomer based on an asymmetric carbon existing at the position where the ring F is substituted, etc., and exists as R-isomer, S-isomer or a mixture thereof in connection with individual asymmetric carbon. It can be separated into individual R-isomer and S-isomer with usual optical resolution and individual optical isomers and a mixture thereof are included by Compound (III). For example, Compound (III) includes the following individual optical isomers and a mixture thereof.

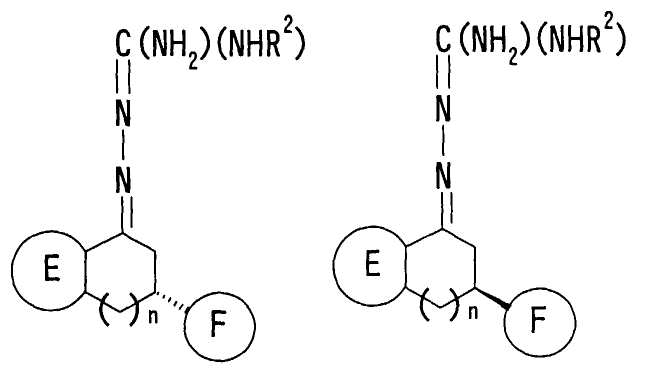

In addition, Compound (III) is equivalent to Compound (IIIa) and Compound (IIIb) in view of its chemical structure.

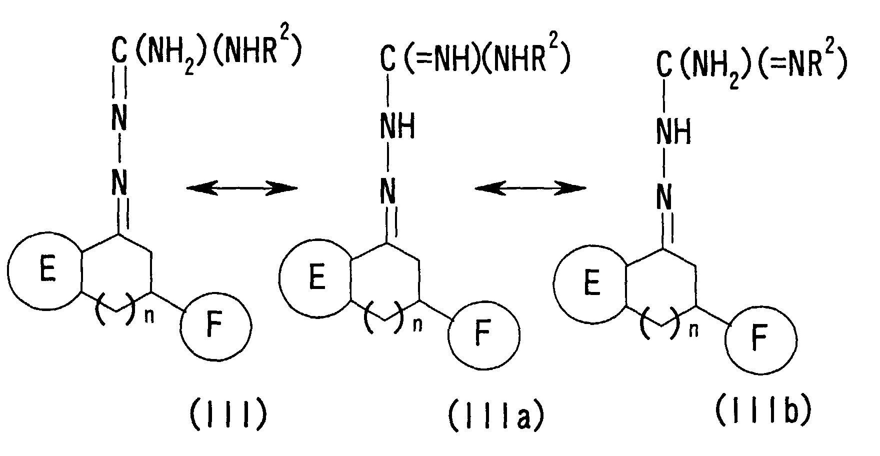

In the present invention, the compound having endothelin antagonistic activity or a salt thereof [hereinafter, referred to as ET antagonist] and the compound having activity for inhibiting Na-H exchange or a salt thereof [hereinafter, referred to as Na-H exchange inhibitor] may be independently pro-drug.

The pro-drug of the ET antagonist or the Na-H exchange inhibitor means a compound which is converted to the ET antagonist or the Na-H exchange inhibitor under the physiological condition or with a reaction due to an enzyme, an gastric acid, etc. in the living body, that is, a compound which is converted to the ET antagonist or the Na-H exchange inhibitor with oxidation, reduction, hydrolysis, etc. according to an enzyme; a compound which is converted to the ET antagonist or the Na-H exchange inhibitor with gastric acid, etc.; etc.

Examples of the pro-drug of the ET antagonist or the Na-H exchange inhibitor include a compound wherein an amino group of the ET antagonist or the Na-H exchange inhibitor is substituted with acyl, alkyl, phosphoric acid, etc. (e.g. a compound wherein an amino group of the ET antagonist or the Na-H exchange inhibitor is substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc.); a compound wherein an hydroxy group of the ET antagonist or the Na-H exchange inhibitor is substituted with acyl, alkyl, phosphoric acid, boric acid, etc. (e.g. a compound wherein an hydroxy group of the ET antagonist or the Na-H exchange inhibitor is substituted with acetyl, palmitoyl, propanoyl, pivaloyl, succinyl, fumaryl, alanyl, dimethylaminomethylcarbonyl, etc.); a compound wherein a carboxyl group of the ET antagonist or the Na-H exchange inhibitor is modified with ester, amide, etc. (e.g. a compound wherein a carboxyl group of the ET antagonist or the Na-H exchange inhibitor is modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); etc. These pro-drug can be produced by per se known method from the ET antagonist or the Na-H exchange inhibitor.

The pro-drug of the ET antagonist or the Na-H exchange inhibitor may be a compound which is converted into the ET antagonist or the Na-H exchange inhibitor under the physiological conditions as described in "Pharmaceutical Research and Development", Vol. 7 (Drug Design), pages 163-198 published in 1990 by Hirokawa Publishing Co. (Tokyo, Japan).

In the present invention, the compound having endothelin antagonistic activity or a salt thereof is used in combination with the compound having activity for inhibiting Na-H exchange or a salt thereof. The endothelin antagonist can be used in combination with other drugs such as an anticoagulant, a thrombolytic agent, an agent for the prevention of reobstruction or restenosis of infarct of myocardial infarction, an anti-arrhythmia agent, etc. (preferably, an agent for the treatment of myocardial infarction) instead of the above-mentioned compound having activity for inhibiting Na-H exchange. In addition, these drugs may be used in combination with the compound having endothelin antagonistic activity or a salt thereof and the compound having activity for inhibiting Na-H exchange or a salt thereof.

To state further, in the case of using the compound having endothelin antagonistic activity or a salt thereof in combination with the compound having activity for inhibiting Na-H exchange or a salt thereof, these drugs can be formulated by mixing individually or simultaneously with pharmaceutically acceptable carriers, excipients, binders, diluents, etc., which can be administered orally or non-orally as a pharmaceutical composition. In the case of formulating these effective components individually, while the individually formulated agents can be administered in the form of their mixture prepared by using e.g. a diluent when administered, the individually formulated agents can also be administered separately or simultaneously or with time intervals to the one and same subject. A kit for administering the individually formulated effective components in the form of their mixture prepared by using e.g. a diluent when administered (e.g. a kit for injection which comprises two or more ampoules each comprising a powdery component and a diluent for mixing and dissolving two or more components when administered, etc.), a kit for administering the individually formulated agents simultaneously or with time intervals to the one and the same subject (e.g. a kit for tablets to be administered simultaneously or with time intervals, characterized by having two or more tablets each comprising an agent and said tablets being put in one or separate bags and, if necessary, a column to describe the time each agent is to be administered, etc.), etc. are also included by the pharmaceutical composition of the present invention.

As the pharmaceutical composition of the present invention, a preferable combination includes, for example, disodium salt of Cyclo[-D-Asp-Asp(R1)-Asp-D-Thg(2)-Leu-D-Trp-], in which Asp(R1) is aspartic acid β-4-phenylpiperazineamide residue and Thg(2) is 2-thienylglycine residue, and cariporide, etc., but the present invention should not be restricted to said combination.

The pharmaceutical composition of the present invention for the prevention or treatment of ischemic disease exhibits excellent cell disorder ameliorating activity or cell-protecting activity in animals, especially mammals (for example, human, monkey, swine, dog, cat, rabbit, guinea pig, rat, mouse, etc.) and is useful as an agent for the prevention or treatment of ischemic disease (for example, ischemic cardiac disease, etc. such as myocardial infarction and dysfunctions accompanying thereto, unstable angina, etc.), reocclusion after PTCA, arrhythmia, cardiac insufficiency, hypercardia, hypertension and tissue disorders accompanying thereto, ischemic encephalic disease (for example, cerebral infarction, cerebral hemorrhage, cerebral disorders accompanying to subarachnoid hemorrhage, etc.), ischemic renal disease, ischemic liver disease, hypofunction of an organ (for example, liver, kidney, heart, lung, spleen, pancreas, etc.) after surgery or transplantation of said organ, etc. (preferably, an agent for the prevention or treatment of ischemic cardiac disease such as myocardial infarction and dysfunctions accompanying thereto, unstable angina, etc., reocclusion after PTCA, arrhythmia, cardiac insufficiency, hypercardia, etc.; more preferably an agent for the prevention or treatment of ischemic cardiac disease such as myocardial infarction, etc., cardiac insufficiency, etc.). Here, conception of the prevention of cardiac insufficiency includes the treatment of prognosis of myocardial infarction (inhibition of enlargement of infarct, prevention of change to cardiac insufficiency, etc.). Also, conception of the treatment of cardiac insufficiency includes inhibition of evolution or grave of cardiac insufficiency, etc.

The pharmaceutical composition of the present invention for the prevention or treatment of ischemic disease is of low toxicity and can be orally or non-orally safely administered as such, or as pharmaceutical compositions such as powders, granules, tablets, capsules (including soft capsules, microcapsule), liquid preparations, injections, suppositories, etc. in combination with an appropriate pharmaceutically acceptable carrier, excipient, diluent, etc., when used as an agent as described above.

The pharmaceutical composition of the present invention for the prevention or treatment of ischemic disease can be prepared as pharmaceutical preparations by ordinary methods.

In the present specification, "non-oral" includes subcutaneous injection, intravenous injection, intramuscular injection, intraperitoneal injection, drip infusion, etc.

Injectable preparations, for example, aqueous or oily suspensions for aseptic injection, can be prepared by methods known in relevant fields, using an appropriate dispersing agent or wetting agent and a suspending agent. The aseptic injectable preparation may be an aseptically injectable solution or suspension in a diluent or solvent which permits non-toxic non-oral administration, such as an aqueous solution, etc. Acceptable vehicles or solvents include water, Ringer's solution, isotonic saline, etc. It is also possible to use aseptic non-volatile oils in common use as solvents or suspending media. For this purpose, any non-volatile oil or fatty acid can be used, including natural, synthetic or semi-synthetic fatty oils or fatty acids, and natural, synthetic or semi-synthetic mono-, di- or tri-glycerides.

Suppositories for rectal administration may be produced as a mixture of the drug and an appropriate non-irritative shaping agent, such as cacao butter or polyethylene glycol, which is solid at normal temperatures and which is liquid at intestinal temperatures and melts and releases the drug in the rectum.

Solid dosage form for oral administration include the above-mentioned forms such as powders, granules, tablets, pills, capsules, etc. In these dosage form, the active ingredient compound may be mixed with at least one additive such as sucrose, lactose, cellulose sugar, mannitol, maltitol, dextran, starch, agar, alginate, chitin, chitosan, pectin, gum traganth, gum arabic, gelatin, collagen, casein, albumin, synthetic or semi-synthetic polymer or glyceride. Such dosage forms may contain the usual additional additives, including inert diluents, lubricants such as magnesium stearate, etc., preservatives such as paraben, sorbic acid, etc., antioxidants such as ascorbic acid, α-tocopherol, cysteine, etc., disintegrating agents, binders, thickening agents, buffers, sweeteners, flavoring agents, perfumes, etc. Tablets and pills may be produced with enteric coating.

Liquid dosage forms for oral administration include pharmaceutically acceptable emulsions, syrups, elixirs, suspensions, solutions, etc., which may contain inert diluents, such as water, in common use in relevant fields.

The dose of the pharmaceutical composition of the present invention for the prevention or treatment of ischemic disease is determined in accordance with the minimal recommendable clinical dose of individual drugs, and can be selected dependent on the subject, age, body weight, symptom, dose interval, administration route, type of formulation, combination of drugs, etc.

The dose to be administered to a specific patient is dependent on the age, body weight, general health conditions, sex, diet, dose interval, administration route, excretion rate, combination of drugs, conditions of the disease then treated, other factors, etc.

Typical daily dose of combination of the compound having endothelin antagonistic activity or a salt thereof with the compound having activity for inhibiting Na-H exchange or a salt thereof is within the range of from about 1/50 of the minimal recommendable clinical dose to the maximum recommendable clinical dose (preferably from minimal recommendable clinical dose or less, more preferably 1/2 of minimal recommendable clinical dose or less) in the case of practical administration of these compounds individually.

For example, in case of the treatment of myocardial infarction in human adult (body weight: about 60kg) by oral administration, a single dose of the compound having activity for inhibiting Na-H exchange or a salt thereof usually ranges from about 0.002 to about 5mg/kg, preferably from 0.005 to 2mg/kg, more preferably from 0.02 to 1mg/kg, and it is desirable that such dosage is given about once to about 3 times a day, depending on symptoms. In acute onset of disease, for example, just after myocardial infarction, higher dose and especially higher dosing frequencies, for example, 4 times a day, may be necessary. In particular, in the case of a patient with myocardial infarction under intensive care treatment, about 50mg/patient per day may be necessary for intravenous administration.

On the other hand, the dose ranging from about 10-300mg/human/day (preferably, about 20-200mg/human/day, more preferably, about 50-100mg/human/day) of the endothelin antagonist (preferably by intravenous administration) is effectively combined with the Na-H exchange inhibitor. Needless to state, while these dosage ranges can be adjusted by a necessary unit base for dividing a daily dose, such doses are decided by taking into consideration the diseases to be treated, conditions of such disease, the age, body weight, general health conditions, sex, diet, dose intervals, administration route, excretion rate, combination of drugs, and other factors. Embodiments of the combination use of the endothelin antagonist with the Na-H exchange inhibitor include a method for administering the Na-H exchange inhibitor continuously by drip infusion or by oral administration before myocardial infarction or after myocardial infarction and administering the endothelin antagonist just after myocardial infarction, etc.

### Best mode for carrying out the invention

The present invention is hereinafter described in more detail by means of the following Experimental Examples and Reference Examples which are not to be construed as limitative.

### Examples

### Experimental Example 1

Rat coronary is obstructed for 1 hour and then blood perfusion is started again to cause myocardial infarction. For 8-20 weeks during experiment period, various parameters relating to myocardial infarction and cardiac insufficiency are measured. Either endothelin antagonist or NaH exchange inhibitor alone, or both of them is administered before obstruction.

### Embodiment (1):

Compound A (0.1-3mg/kg) as endothelin antagonist is intravenously administered before obstruction or before blood reperfusion, and NaH exchange inhibitor (a compound of the formula (III) or a salt thereof) (1-30mg/kg) is orally administered once a day after blood reperfusion.

### Embodiment (2):

Compound A and NaH exchange inhibitor (a compound of the formula (III) or a salt thereof) are simultaneously administered before obstruction or before blood reperfusion, and the NaH exchange inhibitor is administered once a day after blood reperfusion.

### Experimental Example 2

(1) Inhibitory effects of Compound A on the extension of myocardial infarct size were determined by "Rat acute myocardial infarction model" described in British Journal of Pharmacology, 114, 949-954 (1995).
The results are shown in the following Table.

| Pre-treatment with Compound A (1 mg/kg, i.v.; 10 minutes before coronary occlusion) | |
|---|---|
| Drug | infarct size/area-at-risk |
| Saline (n=12) | 60±2 % |
| Compound A (n=7) | 41±3 % *** |

| Post-treatment with Compound A (1 mg/kg, i.v.; 10 minutes before reperfusion started) | |
|---|---|
| Drug | infarct size/area-at-risk |
| Compound A (n=12) | 40±2 % *** |

| | |
|---|---|
| *** P<0.001 vs vehicle | |

(2) Inhibitory effects of Compound B [(S)-(-)-7-(2-chlorophenyl)-5-guanidinoimino-4-methyl-5,6,7,8-tetrahydroquinoline] on the extension of myocardial infarct size were determined by "Rat acute myocardial infarction model" described in British Journal of Pharmacology, 114, 949-954 (1995).
The results are shown in the following Table.

| Pre-treatment with Compound B (1 mg/kg, i.v.; 5 minutes before coronary occlusion) | |
|---|---|
| Drug | infarct size/area-at-risk |
| Saline (n=5) | 67±2 % |
| Compound B (n=5) | 30±5 % t** |

| Post-treatment with Compound B (1 mg/kg, i.v.; 1 minute before reperfusion started) | |
|---|---|
| Drug | infarct size/area-at-risk |
| Saline (n=6) | 63±3 % |
| Compound B (n=6) | 39±3 % ** |

| | |
|---|---|
| ** P<0.01 vs vehicle | |

### Experimental Example 3

Inhibitory effects of Compounds A and B on lethal arrhythmia were determined by the following method:

Male Wistar rats (10-week-old) were anaesthetized with pentobarbital and the chest was opened to expose the heart. Arrhythmia was induced by coronary artery occlusion for 5 minutes with reperfusion of the coronary blood flow. Ventricular arrhythmia observed during occlusion and for 5 minutes after reperfusion was recorded on Mac Lab through polygraph, and the II Lead electro-cardiogram was recorded. Ventricular tachycardia (VT) was counted when premature ventricular complex (PVC) continued more than 5 times. Each of the drugs was dissolved in saline and administered from the femoral vein 5 minutes before coronary artery occlusion.

The results are shown in the following Table.

| Drug | VT | | PVC | |
|---|---|---|---|---|
| | D (second) | F (time) | D (second) | N (time) |
| Saline (n=6) | 77.4±38.0 | 9.5±2.1 | 69.4±7.6 | 33.7±9.5 |
| Cpd A (n=9) | 66.8±36.4 | 7.6±1.2 | 51.4±8.5 | 33.4±10.1 |
| Cpd B (n=6) | 7.2±3.6 | 3.0±0.7* | 20.9±4.3** | 4.8±2.8* |
| Cpd=Compound; F=frequency; D=duration; N=number of PVCs | | | | |

| | | | | |
|---|---|---|---|---|
| * P<0.05 vs vehicle | | | | |
| ** P<0.01 vs vehicle | | | | |

As shown above, Compound B showed inhibitory effects on lethal arrhythmia, while Compound A did not show inhibitory effects on lethal arrhythmia. Therefore, combination of Compound A with Compound B can provide an effective drug for both of myocardial infarction and lethal arrhythmia.

### Synthesis of (S)-(-)-7-(2,5-dichlorothiophen-3-yl)-5-guanidinoimino-4-methyl-5,6,7,8-tetrahydroquinoline [Compound 1] and (±)-7-(2,5-dichlorothiophen-3-yl)-5-guanidinoimino-4-methyl-5,6,7,8-tetrahydroquinoline [Compound 2]

### Reference Example 1

To a solution of 2,5-dichlorothiophene (100.0g) and dichloromethylmethylether (165.3g) in dichloromethane (800ml) was added dropwise a solution of titanium tetrachloride (272.7g) in dichloromethane (160ml) at -10 to -15°C taking 50 minutes, and at the same temperature, the mixture was stirred for 30 minutes. The reaction solution was poured into ice, and the organic layer was washed with water, sodium hydrogen carbonate solution, water and saturated brine, and dried with magnesium sulfate. Under reduced pressure, the solvent was evaporated to give 2,5-dichloro-3-formylthiophene (115.0g) as oil. To a mixture of acetone (1000ml), sodium hydroxide (28.6g) and water (1200ml) was added at 0°C a solution of 2,5-dichloro-3-formylthiophene (58.6g) in acetone (200ml) for 1.5 hours, and the mixture was stirred at the same temperature for 1 hour. Under reduced pressure, acetone was evaporated, and the crystals were filtered, washed with water and dried to give 4-(2,5-dichlorothiophen-3-yl)-3-buten-2-one (136.6g). To a solution of 20% sodium ethoxide in ethanol (211g) was added diethyl malonate (99.3g) at room temperature, and then 4-(2,5-dichlorothiophen-3-yl)-3-buten-2-one(136.6g) little by little. The mixture was stirred at room temperature for 30 minutes, heated for 2 hours while stirring, and cooled. The solvent was evaporated, and to the residue was added water. The aqueous layer was washed with ethyl acetate concentrated, to which was added 2M sodium hydroxide (340ml). The mixture was stirred at 100°C for 2 hours and cooled. To the mixture was added 2.5M sulfuric acid (340ml) for 15 minutes, and the mixture was stirred at 100°C for 1.5 hours. The mixture was cooled, and precipitated crystals were filtered and washed with ethyl acetate-isopropylether (1:4) and isopropylether to give 5-(2,5-dichlorothiophen-3-yl)cyclohexane-1,3-dione(78.9g) as colorless crystals.
mp 200°C (decomp.)
¹H-NMR(CDCl₃)δ: 2.36-2.61 (4H, m), 3.42-3.62 (1H, m), 5.51 (1H, s), 6.76 (1H, s), 8.0-12.5 (1H, br).

### Reference Example 2

A solution of 5-(2,5-dichlorothiophen-3-yl)cyclohexane-1,3-dione (42.0g) and ammonium acetate (36.9g) in ethanol (840ml) was refluxed for 12 hours. Under reduced pressure, the solvent was evaporated, and to the residue was added water. The crystals were filtered, washed with toluene and dried to give 1-amino-5-(2,5-dichlorothiophen-3-yl)cyclohexen-3-one (40.3g). To a solution of 1-amino-5-(2,5-dichlorothiophen-3-yl)cyclohexen-3-one (37.0g) in ethanol (700ml) and toluene (1400ml) were added 3-oxobutylaldehydedimethylacetal (46.6g) and powdery potassium hydroxide (7.7g), and the mixture was refluxed. To the mixture was added powdery potassium hydroxide (1.6g) 30 minutes later; powdery potassium hydroxide (1.6g) and 3-oxobutylaldehydedimethylacetal (3.7g) 1 hour later; and powdery potassium hydroxide (1.6g) 1.5 hours later; and then the mixture was stirred at the same temperature for 2 hours. After the mixture was cooled, the solvent was evaporated under reduced pressure and to the residue was added ethyl acetate. The mixture was washed with water and saturated brine, and dried with magnesium sulfate. Under reduced pressure, ethyl acetate was evaporated, and the thus obtained oil containing crystals were subjected to silica gel column (EtOAc/hexane) to remove starting materials. The thus obtained crystals were recrystallized from ethyl acetate-hexane to give 7-(2,5-dichlorothiophen-3-yl)-4-methyl-5,6,7,8-tetrahydroquinoline-5-one (32.6g).
mp. 138-140°C.
¹H-NMR(CDCl₃)δ: 2.70 (3H, s), 2.75 (1H, dd, J=12, 17Hz), 2.93 (1H, ddd, J=2, 4, 16Hz), 3.22 (1H, dd, J=11, 17Hz), 3.39 (1H, ddd, J=2, 5, 17Hz), 3.57-3.76 (1H, m), 6.72 (1H, s), 7.11 (1H, d, J=5Hz), 8.50 (1H, d, J=5Hz).

### Reference Example 3

A mixture of 7-(2,5-dichlorothiophene-3-yl)-4-methyl-5,6,7,8-tetrahydroquinoline-5-one (218 mg), aminoguanidine hydrochloride (83 mg) and concentrated hydrochloric acid (0.1 ml) in ethanol (4 ml) was stirred at 100 °C (bath temperature) for 14 hours. The reaction solution was cooled, and precipitated crystals were filtered, washed with ethanol and dried to give 7-(2,5-dichlorothiophene-3-yl)-5-guanidinoimino-4-methyl-5,6,7,8-tetrahydroquinolinehydrochloride (250mg) as pale yellow crystals.
mp. 300 °C or more

| Elemental Analysis for C₁₅H₁₅N₅Cl₂S·2HCl | | | |
|---|---|---|---|
| Calcd. | C, 40.83; | H, 3.88; | N, 15.87 |
| Found | C, 40.75; | H, 3.64; | N, 15.69. |

¹H-NMR(DMSO-d₆) δ: 2.85 (4H, m), 3.12 (1H, dd), 3.36 (3H, m), 7.42 (1H, s), 7.79 (1H, d), 7.90 (4H, broad), 8.62 (1H, d), 11.44 (1H, broad).

### Reference Example 4

In methanol (390ml) was suspended (±)-7-(2,5-dichlorothiophen-3-yl)-5-guanidinoimino-4-methyl-5,6,7,8-tetrahydroquinoline hydrochloride (39.0g), and to the suspension was added dropwise a solution of 28% sodium methoxide in methanol. The mixture was stirred at 50°C for 1 hour. Under reduced pressure, the mixture was concentrated, and the obtained crystals were washed with water and dried to give (±)-7-(2,5-dichlorothiophen-3-yl)-5-guanidinoimino-4-methyl-5,6,7,8-tetrahydroquinoline (32.8g).
mp.250-251°C

| Elemental Analysis for C₁₅H₁₅Cl₂N₅S·0.2H₂O | | | |
|---|---|---|---|
| Calcd. | C,48.45; | H,4.17; | N,18.83. |
| Found | C,48.38; | H,4.40; | N,18.74. |

¹H-NMR(CD₃OD) δ: 2.70 (3H, s), 2.74 (1H, dd, J=13, 18Hz), 3.02-3.08 (2H, m), 3.13-3.38 (2H, m), 6.97 (1H, s), 7.19 (1H, d, J=5Hz), 8.14 (1H, d, J=5Hz).

### Reference Example 5

Using CHIRALPAK AD (eluent: hexane-ethanol), (±)-7-(2,5-dichlorothiophen-3-yl)-4-methyl-5,6,7,8-tetrahydroquinoline-5-one (1.0g) was subjected to optical resolution to give (-)-7-(2,5-dichlorothiophen-3-yl)-4-methyl-5,6,7,8-tetrahydroquinoline-5-one (0.40g) and (+)-7-(2,5-dichlorothiophen-3-yl)-4-methyl-5,6,7,8-tetrahydroquinoline-5-one (0.41g).

In ethanol (10ml) was dissolved (-)-7-(2,5-dichlorothiophen-3-yl)-4-methyl-5,6,7,8-tetrahydroquinoline-5-one (0.35g), and to the mixture were added aminoguanidine hydrochloride (0.15g), concentrated hydrochloric acid (0.28ml) and water (0.28ml). The mixture was refluxed for 4 hours, and under reduced pressure, the solvent was evaporated. The residue was dissolved in water, and the mixture was washed with ethyl acetate and concentrated under reduced pressure. The residue was heated in a little amount of ethanol, and the solution was cooled. Precipitated crystals were filtered off, and the mother liquor was concentrated to give crystals, which were recrystallized from water to give (-)-7-(2,5-dichlorothiophen-3-yl)-5-guanidinoimino-4-methyl-5,6,7,8-tetrahydroquinoline hydrochloride (0.47g) as colorless crystals.

| Elemental Analysis for C₁₅H₁₅C₁₂N₅S·2HCl·H₂O | | | | |
|---|---|---|---|---|
| Calcd. | C,39.23; | H,4.17; | N,15.25; | Cl,30.88. |
| Found | C,39.06; | H,4.31; | N,15.25; | Cl,30.69. |

¹H-NMR(DMSO-d₆) δ: 2.85 (4H, m), 3.12 (1H, dd), 3.36 (3H, m), 7.42 (1H, s), 7.79 (1H, d), 7.90 (4H, broad), 8.62 (1H, d), 11.44 (1H, broad).

In ethanol (10ml) was dissolved (+)-7-(2,5-dichlorothiophen-3-yl)-4-methyl-5,6,7,8-tetrahydroquinoline-5-one (0.36g), and to the mixture were added aminoguanidine hydrochloride (0.15g), concentrated hydrochloric acid (0.29ml) and water (0.29ml). The mixture was refluxed for 4 hours, and under reduced pressure, the solvent was evaporated. The residue was dissolved in water, and the mixture was washed with ethyl acetate and concentrated under reduced pressure. The residue was heated in a little amount of ethanol, and the solution was cooled. Precipitated crystals were filtered off, and the mother liquor was concentrated to give crystals, which were recrystallized from water to give (+)-7-(2,5-dichlorothiophen-3-yl)-5-guanidinoimino-4-methyl-5,6,7,8-tetrahydroquinoline hydrochloride (0.46g) as colorless crystals.

| Elemental Analysis for C₁₅H₁₅C₁₂N₅S·2HCl·0.5H₂O | | | |
|---|---|---|---|
| Calcd. | C,40.02; | H,4.03; | N,15.56. |
| Found | C,39.69; | H,4.17; | N,15.50. |

¹H-NMR(DMSO-d₆) was agreed with that of the above free compound.

### Reference Example 6

To a solution of (±)-7-(2,5-dichlorothiophen-3-yl)-5-guanidinoimino-4-methyl-5,6,7,8-tetrahydroquinoline (17.4g) in ethanol (200ml) was added a solution of L-pyroglutamic acid (4.0g) in ethanol (20ml), at 80°C, and the mixture was gradually cooled to the room temperature and stirred at room temperature for 6 hours. The crystals were filtered and washed with ethanol to give (+)-7-(2,5-dichlorothiophen-3-yl)-5-guanidinoimino-4-methyl-5,6,7,8-tetrahydroquinoline L-pyroglutamate (11.0g). Said crystals were suspended in methanol (200ml), and to the mixture was added a solution of 28% sodium methoxide in methanol (4.2ml). Under reduced pressure, the solvent was evaporated to give crystals, which were washed with water, dried and recrystallized from ethanol. To the crystals was added ethanol (30ml), and then methanesulfonic acid (3.4g). The mixture was heated to give a homogenous solution and cooled, and precipitated crystals were filtered to give (+)-7-(2,5-dichlorothiophen-3-yl)-5-guanidinoimino-4-methyl-5,6,7,8-tetrahydroquinoline methanesulfonate (8.5g, 99.9 % ee).
mp. 225-229°C

| Elemental Analysis for C₁₅H₁₅Cl₂N₅S·2MeSO₃H | | | | |
|---|---|---|---|---|
| Calcd. | C,36.43; | H,4.14; | N,12.49; | Cl,12.65. |
| Found | C,36.61; | H,4.14; | N,12.39; | Cl,12.57. |

¹H-NMR(DMSO-d₆) δ: 2.41 (6H, s), 2.70-2.94 (1H, m), 2.86 (3H, s), 2.97-3.26 (2H, m), 3.27-3.57 (2H, m), 7.2-8.4 (4H, br), 7.41 (1H, s), 7.82 (1H, d, J=6Hz). 8.65 (1H, d, J=6Hz), 10.80 (1H, s).

To the mother liquor obtained by the separation treatment with L-pyroglutamic acid and washing solution was added a solution of 28% sodium methoxide in methanol (3ml), and the mixture was concentrated and washed with water to give (-)-isomer rich crystals (9.3g,78.5 % ee), which were recrystallized from ethanol to give (-)-7-(2,5-dichlorothiophen-3-yl)-5-guanidinoimino-4-methyl-5,6,7,8-tetrahydroquinoline (7.6g,99.2% ee).
(This compound was confirmed to be S-isomer (absolute configuration), according to X-ray crystal structure analysis.)
mp.129-133°C
¹H-NMR(CD₃OD) δ: 2.70 (3H, s), 2.74 (1H, dd, J=13, 18 Hz), 3.02-3.08 (2H, m), 3.13-3.38 (2H, m), 6.97 (1H, s), 7.19 (1H, d, J=5Hz), 8.14 (1H, d, J=5Hz)

To (-)-7-(2,5-dichlorothiophen-3-yl)-5-guanidinoimino-4-methyl-5,6,7,8-tetrahydroquinoline (6.4g) was added ethanol (50ml) and methanesulfonic acid (3.1g) to give a homogenous solution, and the solution was concentrated to give crystals, which were recrystallized from ethanol to give (-)-7-(2,5-dichlorothiophen-3-yl)-5-guanidinoimino-4-methyl-5,6,7,8-tetrahydroquinoline methanesulfonate (8.1g,99.5 % ee).
mp. 229-231°C

| Elemental Analysis for C₁₅H₁₅Cl₂N₅S·2MeSO₃H | | | | |
|---|---|---|---|---|
| Calcd. | C,36.43; | H,4.14; | N,12.49; | Cl,12.65. |
| Found | C,36.50; | H,4.06; | N,12.34; | Cl,12.62. |

¹H-NMR(DMSO-d₆) was agreed with that of the above free compound.

### Reference Example 7

To a solution of (-)-7-(2,5-dichlorothiophen-3-yl)-5-guanidinoimino-4-methyl-5,6,7,8-tetrahydroquinoline (0.8g) in ethanol (20ml) was added methanesulfonic acid (0.37g), and the mixture was concentrated under reduced pressure. To the residue was added water (1ml), and the mixture was concentrated to precipitate crystals, which were filtered and washed with ethanol. The thus obtained crystals were dried to give (-)-7-(2,5-dichlorothiophen-3-yl)-5-guanidinoimino-4-methyl-5,6,7,8-tetrahydroquinoline methanesulfonate monohydrate (1.0g).
mp. 239-241°C

| Elemental Analysis for C₁₅H₁₅Cl₂N₅S·2MeSO₃H·H₂O | | | |
|---|---|---|---|
| Calcd. | C,35.29; | H,4.36; | N,12.11. |
| Found | C,35.11; | H,4.27; | N,12.15. |

¹H-NMR(DMSO-d₆) δ: 2.41 (6H, s), 2.70-2.94 (1H, m), 2.86 (3H, s), 2.97-3.26 (2H, m), 3.27-3.57 (2H, m), 7.2-8.4 (4H, br), 7.40 (1H, s), 7.83 (1H, d, J=6Hz), 8.65 (1H, d, J=6Hz), 10.79 (1H, s).

### Reference Example 8

To a solution of (-)-7-(2,5-dichlorothiophen-3-yl)-5-guanidinoimino-4-methyl-5,6,7,8-tetrahydroquinoline (0.8g) in ethanol (20ml) was added 0.5M sulfuric acid (3.9ml), and the mixture was concentrated under reduced pressure to give crystals, which were recrystallized from water and washed with ethanol to give (-)-7-(2,5-dichlorothiophen-3-yl)-5-guanidinoimino-4-methyl-5,6,7,8-tetrahydroquinoline sulfurate (0.8g).
mp. 239-242°C

| Elemental Analysis for C₁₅H₁₅ Cl₂N₅S·H₂SO₄·0.5H₂O | | | |
|---|---|---|---|
| Calcd. | C,37.90; | H,3.82; | N,14.73. |
| Found | C,37.87; | H,3.88; | N,14.56. |

¹H-NMR(DMSO-d₆) δ: 2.40-3.6 (5H, m), 2.64 (3H, s), 7.0-8.0 (4H, br), 7.37 (1H, s), 7.24 (1H, d, J=5Hz), 8.32 (1H, d, J=5Hz), 10.58 (1H, br).

### Reference Example 9

To a solution of (-)-7-(2,5-dichlorothiophen-3-yl)-5-guanidinoimino-4-methyl-5,6,7,8-tetrahydroquinoline (0.8g) in ethanol (20ml) was added 1.7M nitric acid (2.3ml), and the mixture was concentrated under reduced pressure to give crystals, which were recrystallized from water to give (-)-7-(2,5-dichlorothiophen-3-yl)-5-guanidinoimino-4-methyl-5,6,7,8-tetrahydroquinoline nitrate (0.92g).
mp. 167°C (decomp.)

| Elemental Analysis for C₁₅H₁₅Cl₂N₅S·HNO₃·0.5H₂O | | | |
|---|---|---|---|
| Calcd. | C,35.79; | H,3.60; | N,19.48. |
| Found | C,35.56; | H,3.58; | N,19.39. |

¹H-NMR(DMSO-d₆) δ: 2.69-3.21 (3H, m), 2.83 (3H, s), 3.26-3.63 (2H, m), 7.2-8.0 (4H, br), 7.40 (1H, s), 7.77 (1H, d, J=6Hz), 8.64 (1H, d, J=6Hz), 10.61 (1H, s).

### Synthesis of (S)-(-)-7-(2-chlorophenyl)-5-guanidinoimino-4-methyl-5,6,7,8-tetrahydroquinoline [Compound 3] and (±)-7-(2-chlorophenyl)-5-guanidinoimino-4-methyl-5,6,7,8-tetrahydroquinoline [Compound 4]

### Reference Example 10

To a mixed solution of acetone (294 ml) and an aqueous solution (1.41 ml) of sodium hydroxide (22.0 g) was added 2-chlorobenzaldehyde (70.3 g), and the mixture was stirred at room temperature for 5 hours. Excess acetone was evaporated under reduced pressure, and to the residue was added ethyl acetate (1.4 l). The mixture was subjected to extraction, and the ethyl acetate layer was washed with brine and dried (anhydrous magnesium sulfate). Under reduced pressure, ethyl acetate was evaporated to give crude 2-chlorobenzalacetone (94.6 g) as yellow oil, which was used for the following reaction without further purification.

To a solution of 20 % sodium ethoxide in ethanol (170.1 g) was added diethyl malonate (80.1 g) at room temperature (immediately precipitated material was observed), and then a solution of crude 2-chlorobenzalacetone (94.6 g) in ethanol (40 ml). The mixture was stirred at 90 °C for 2 hours, cooled at room temperature and under-ice-cooling (for 1 hour). Precipitated materials were filtered and washed with ethyl acetate and isopropylether to give crude ethyl 6-(2-chlorophenyl)-2-hydroxy-4-oxo-2-cyclohexene-1-carboxylate monosodium salt (151.0 g) as pale yellow powder. To said powder was added 2 M sodium hydroxide (350 ml), and the mixture was stirred at 100 °C for 2 hours and cooled. To the mixture was added 2.5 M sulfuric acid (350 ml) for 15 minutes, and the mixture was stirred at 100 °C for 2 hours and cooled. To the mixture was added ethyl acetate (1.4 l), and the mixture was subjected to extraction. The ethyl acetate layer was washed with brine, dried (anhydrous magnesium sulfate), and ethyl acetate was evaporated under reduced pressure. Precipitated crystals were washed with ethyl acetate-isopropylether (1:4) and isopropylether to give 5-(2-chlorophenyl)cyclohexane-1,3-dione (82.1 g) as colorless crystals.
mp 157-158 °C.

### Reference Example 11

A solution of 5-(2-chlorophenyl)cyclohexane-1,3-dione (2.5 g) and ammonium acetate (2.6 g) in ethanol (50 ml) was refluxed for 12 hours. Under reduced pressure, the solvent was evaporated, and to the residue was added sodium hydrogen carbonate solution. The mixture was extracted with ethyl acetate, and the organic layer was washed with water and saturated brine, and dried with magnesium sulfate. Under reduced pressure, the solvent was evaporated to give crystals, which were recrystallized from ethyl acetate-hexane to give pale yellow crystals of 1-amino-5-(2-chlorophenyl)cyclohexen-3-one (2.2 g).
mp. 199 °C (decomp.)
¹H-NMR(CDCl₃) δ: 2.44-2.72 (4H, m), 3.77-3.97 (1H, m), 4.68 (2H, br), 5.35 (1H, s), 7.15-7.43 (4H, m).

### Reference Example 12

To a mixture of 5-(2-chlorophenyl)-1,3-cyclohexanedione (1.1 g), 1-amino-2-butin hydrochloride (0.5 g), molecular sieves 4A (2 g) and tetrahydrofuran (20 ml) was added triethylamine (0.48 g), and the mixture was stirred at room temperature for 1 hour and then refluxed for 12 hours and cooled. Insoluble materials were filtered, and under reduced pressure, the solvent was evaporated. The residue was stirred for 4 hours at 220 °C, to which were added ethyl acetate and sodium hydrogen carbonate solution. The organic layer was washed with water and saturated brine, and dried with magnesium sulfate. Under reduced pressure, the mixture was concentrated, and the residue was subjected to silica gel column chromatography (EtOAc/hexane) to give crystals, which were recrystallized from ethyl acetate-hexane to give colorless crystals of 7-(2-chlorophenyl)-4-methyl-5,6,7,8-tetrahydroquinoline-5-one (0.20 g).
mp. 97-98 °C
¹H-NMR(CDCl₃) δ: 2.71 (3H, s), 2.84 (1H, dd, J=13, 16 Hz), 3.02 (1H, ddd, J=2, 4, 16 Hz), 3.30 (1H, dd, J=12, 17 Hz), 3.48 (1H, ddd, J=2, 4, 17 Hz), 3.88-4.07 (1H, m), 7.11 (1H, d, J=5 Hz), 7.16-7.34 (4H, m), 8.50 (1H, d, J=5 Hz).

### Reference Example 13

To a solution of 1-amino-5-(2-chlorophenyl)cyclohexen-3-one (2.7 g) in ethanol (50 ml) and toluene (150 ml) were added acetylacetoaldehyde-dimethylacetal (4.0 g) and 85% potassium hydroxide (0.67 g), and the mixture was refluxed. With intervals of 30 minutes, 85% potassium hydroxide (0.14 g) was added 3 times to the mixture. Then, the mixture was refluxed for 1 hour. Under reduced pressure, the solvent was evaporated, and to the residue was added ethyl acetate. The mixture was washed with water and saturated brine, and dried with magnesium sulfate. Under reduced pressure, the mixture was concentrated, and the residue was subjected to silica gel column chromatography (EtOAc-hexane) to give crystals of 7-(2-chlorophenyl)-4-methyl-5,6,7,8-tetrahydroquinoline-5-one (2.5 g).
mp. 97 - 98°C
¹H-NMR(CDCl₃) δ: 2.71 (3H, s), 2.84 (1H, dd, J=13, 16 Hz), 3.02 (1H, ddd, J=2, 4, 16 Hz), 3.30 (1H, dd, J = 12, 17 Hz), 3.48 (1H, ddd, J=2, 4, 17 Hz), 3.88-4.07 (1H, m), 7.11 (1H, d, J=5 Hz), 7.16-7.34 (4H, m), 8.50 (1H, d, J=5 Hz).

### Reference Example 14

A mixture of 7-(2-chlorophenyl)-4-methyl-5,6,7,8-tetrahydroquinoline-5-one (0.20 g), aminoguanidine hydrochloride (0.085g), concentrated hydrochloric acid (0.11 ml), water (0.11 ml) and ethanol (20 ml) was refluxed for 6 hours. Under reduced pressure, the solvent was evaporated, and the residue was dissolved in water. The mixture was washed with ethyl acetate and concentrated under reduced pressure. The residue was recrystallised from ethyl acetate-ethanol to give 7-(2-chlorophenyl)-5-guanidinoimino-4-methyl-5,6,7,8-tetrahydroquinoline hydrochloride (0.21 g) as colorless crystals.
mp. 204 °C(decomp.)

| Elemental Analysis for C₁₇H₁₈N₅Cl·2HCl·0.8H₂O | | | |
|---|---|---|---|
| Calcd. | C, 49.18; | H, 5.24; | N, 16.87. |
| Found | C, 49.46; | H, 5.10; | N, 16.88. |

¹H-NMR(DMSO-d₆) δ: 2.65-3.00 (1H, m), 2.88 (3H, s), 3.15-3.78 (4H, m), 7.2-8.2 (4H, br), 7.28-7.53 (3H, m), 7.58-7.66 (1H, m), 7.83 (1H, d, J=6 Hz), 8.63 (1H, d, J=6 Hz), 11.45 (1H, s).

### Reference Example 15

In methanol (1200 ml) was suspended (±)-7-(2-chlorophenyl)-5-guanidinoimino-4-methyl-5,6,7,8-tetrahydroquinoline hydrochloride (1123.9 g), and to the suspension was added dropwise 28% sodium methoxide in methanol (119.2 ml). The mixture was stirred at 50°C for 30 minutes. Under reduced pressure, the solvent was evaporated, and to the residue was added water. The crystals were filtered, washed with water and dried to give colorless crystals of (±)-7-(2-chlorophenyl)-5-guanidinoimino-4-methyl-5,6,7,8-tetrahydroquinoline (109.3 g).

To a solution of (±)-7-(2-chlorophenyl)-5-guanidinoimino-4-methyl-5,6,7,8-tetrahydroquinoline (109.3 g) in isopropylalcohol (700 ml) was added dropwise a solution of L-pyroglutamic acid (10 g) in isopropylalcohol (700 ml) at 50°C for 1.5 hours, and the mixture was stirred at 50°C for 1 hour and then at room temperature for 2 days. The crystals were filtered and washed with isopropylalcohol to give (-)-7-(2-chlorophenyl)-5-guanidinoimino-4-methyl-5,6,7,8-tetrahydroquinoline L-pyroglutamate (55.5 g, 88%ee), which was recrystallized from ethanol to give crystals of L-pyroglutamate (44.3 g, 97 %ee). The thus obtained crystals of salt was suspended in methanol (500 ml), and to the suspension was added a solution of 28% sodium methoxide in methanol (10.9 mmol). The mixture was stirred at 50°C for 30 minutes, and under reduced pressure, the solvent was evaporated to give crystals, which were washed with water and dried to give (-)-7-(2-chlorophenyl)-5-guanidinoimino-4-methyl-5,6,7,8-tetrahydroquinoline (38.9 g). (This compound was confirmed to be S-isomer (absolute configuration), according to X-ray crystal structure analysis.)

In ethanol (400 ml) was dissolved said (-)-isomer, and to the mixture was added methanesulfonic acid (14.3 g). Under reduced pressure, the solvent was evaporated to give crystals, which were recrystallized from ethanol to give (-)-7-(2-chlorophenyl)-5-guanidinoimino-4-methyl-5,6,7,8-tetrahydroquinoline methanesulfonate (46.8 g, 99.2 %ee).
mp. 194-195°C

| Elemental Analysis for C₁₇H₁₈N₅Cl·2MeSO₃H | | | | |
|---|---|---|---|---|
| Calcd. | C, 43.88; | H, 5.04; | N, 13.47; | Cl, 6.82. |
| Found | C, 43.67; | H, 4.90; | N, 13.18; | Cl, 6.76. |

¹H-NMR(DMSO-d₆) δ: 2.40 (6H, s), 2.78 (1H, dd, J=12, 18 Hz), 2.89 (3H, s), 3.08-3.32 (2H, m), 3.44-3.80 (2H, m), 7.2-8.1 (4H, br), 7.31-7.56 (3H, m), 7.58-7.66 (1H, m), 7.86 (1H, d, J=6 Hz), 8.66 (1H, d, J=6 Hz), 10.77 (1H, s).

### Synthesis of (±)-7-(2,5-dichlorophenyl)-5-guanidinoimino-4-methyl-5,6,7,8-tetrahydrocinnoline [Compound 5]

### Reference Example 16

In water (500ml) was dissolved sodium hydroxide (4.0g), and to the mixture was added acetone (100ml), and then 2,5-dichlorobenzaldehyde (15.9g). The mixture was stirred at room temperature for 1 hour, and acetone was evaporated under reduced pressure. The residue was extracted with ethyl acetate, and the organic layer was washed with water and saturated brine, and concentrated under reduced pressure to give 4-(2,5-dichlorophenyl)-3-buten-2-one (19.0g).

To a solution of 20% sodium ethoxide in ethanol (6.2g) were added at room temperature ethanol (150ml) and diethyl malonate (14.6g), and then added little by little 4-(2,5-dichlorophenyl)-3-buten-2-one (19.0g). The mixture was stirred at room temperature for 30 minutes, refluxed for 2 hours and cooled. The solvent was evaporated, and to the residue was added water. The aqueous layer was washed with ethyl acetate and concentrated, and to the residue was added 2M sodium hydroxide (50ml). The mixture was refluxed for 2 hours and cooled, and to the mixture was added 2.5M sulfuric acid (50ml) for 15 minutes. The mixture was refluxed for 1.5 hours and cooled, and precipitated crystals were filtered and washed with water and toluene to give 5-(2,5-dichlorophenyl)cyclohexane-1,3-dione (9.9g) as colorless crystals.
mp187°C(decomp.).
¹H-NMR(CDCl₃) δ: 2.42-2.70 (4H, m), 3.71-3.89 (1H, m), 5.54 (1H, s), 7.16-7.43 (3H, m).

### Reference Example 17

A mixture of 5-(2,5-dichlorophenyl)cyclohexane-1,3-dione (1.19g), p-toluenesulfonylhydrazide (0.86g) and ethanol (15ml) was refluxed for 2.5 hours and cooled, and precipitated crystals were filtered and washed with ethanol to give 5-(2,5-dichlorophenyl)-1-[2-(4-methylphenylsulfonyl)hydrazino]cyclohexane-3-one (1.67g) as colorless crystals.
mp256-257°C(decomp.).
¹H-NMR(DMSO-d₆) δ:2.23 (1H, dd), 2.40 (3H, s), 2.45-2.65 (3H, m), 3.40-3.55 (1H, m), 5.23 (1H, s), 7.32-7.56 (5H, m), 7.71 (2H, d), 8.00 (1H, br), 9.85 (1H, s).

### Reference Example 18

To a mixture of 5-(2,5-dichlorophenyl)-1-[2-(4-methylphenylsulfonyl)hydrazino]cyclohexane-3-one (1.65g), anhydrous potassium carbonate (0.696g), methanol (10ml) and 1,2-dimethoxyethane (8ml) were added under ice-cooling 1-chloropropan-2-one (0.465g) and sodium iodide (0.15g), and the mixture was stirred at room temperature for 2 hours. To the mixture was added anhydrous potassium carbonate (0.64g), and the mixture was stirred for 3 hours at 80°C. Under reduced pressure, the solvent was evaporated, and to the residue was added ethyl acetate (70ml) and water (30ml). The mixture was shaken, and the separated upper layer was washed with water and concentrated under reduced pressure. The residue was purified with silica gel column chromatography to give 7-(2,5-dichlorophenyl)-4-methyl-5,6,7,8-tetrahydrocinnoline-5-one (0.253g) as yellow brown oil.
¹H-NMR(CDCl₃) δ:2.71 (3H, s), 2.84 (1H, dd), 3.08 (1H, ddd), 3.39 (1H, dd), 3.77 (1H, ddd), 3.87-4.04 (1H, m), 7.22-7.41 (3H, m), 9.16 (1H, s).

### Reference Example 19

To a mixture of 7-(2,5-dichlorophenyl)-4-methyl-5,6,7,8-tetrahydrocinnoline-5-one (246mg) and aminoguanidine hydrochloride (98mg) were added ethanol (5ml) and concentrated hydrochloric acid (0.1ml), and the mixture was stirred at 110°C (bath temperature) for 1.5 hours. The reaction solution was cooled to room temperature, and the crystals were filtered and dried to give 7-(2,5-dichlorophenyl)-5-guanidinoimino-4-methyl-5,6,7,8-tetrahydrocinnoline hydrochloride (215mg) as gray white crystals.
mp256-258°C.

| Elemental Analysis for C₁₆H₁₆N₆Cl₂·2HCl | | | |
|---|---|---|---|
| Calcd. | C,44.06; | H,4.16; | N,19.27. |
| Found | C,43.98; | H,4.18; | N,19.13. |

¹H-NMR(DMSO-d₆) δ: 2.74 (3H, s), 2.93 (1H, dd), 3.15-3.70 (4H, m), 7.44 (1H, dd), 7.56 (1H, d), 7.77 (1H, d), 7.96 (4H, br), 9.28 (1H, s), 11.59 (1H, br).

### Synthesis of (±)-7-(5-chloro-2-methylphenyl)-5-guanidinoimino-4-methyl-5,6,7,8-tetrahydroquinoline [Compound 6]

### Reference Example 20

To a solution of 4-chlorotoluene (25.0g) and dichloromethylmethylether (45.4g) in dichloromethane (160ml) was added dropwise at room temperature a solution of titanium tetrachloride (74.9g) in dichloromethane (40ml), and the mixture was stirred at the same temperature for 15 hours. The reaction solution was poured into ice, and the organic layer was washed with water, sodium hydrogen carbonate solution, water and saturated brine, and dried with magnesium sulfate. Under reduced pressure, the solvent was evaporated, and the residue was subjected to silica gel column chromatography (ethyl acetate-hexane) to give oil of crude 2-chloro-5-methylbenzaldehyde (18.3g) and 5-chloro-2-methylbenzaldehyde (4.1g), respectively.

To a mixture of acetone (160ml), sodium hydroxide (2.6g) and water (160ml) was added dropwise at 0°C a solution of crude 2-chloro-5-methylbenzaldehyde (18.3g) in acetone (30ml), and the mixture was stirred at the same temperature for 1 hour. Under reduced pressure, acetone was evaporated, and the residue was extracted with ethyl acetate. The organic layer was washed with water and saturated brine, and concentrated under reduced pressure to give 4-(2-chloro-5-methylphenyl)-3-buten-2-one (18.9g) as oil.

To a solution of 20% sodium ethoxide in ethanol (4.3g) was added at room temperature diethyl malonate (10.1g), and then added little by little 4-(2-chloro-5-methylphenyl)-3-buten-2-one (18.9g). The mixture was stirred at room temperature for 30 minutes, refluxed for 2 hours and cooled. The solvent was evaporated, and to the residue was added water. The aqueous layer was washed with ethyl acetate and concentrated. To the residue was added 2M sodium hydroxide (33ml), and the mixture was refluxed for 2 hours and cooled. To the mixture was added 2.5M sulfuric acid (33ml) for 15 minutes, and the mixture was refluxed for 30 minutes and cooled. Precipitated crystals were filtered and washed with water and isopropylether to give 5-(2-chloro-5-methylphenyl)cyclohexane-1,3-dione (7.8g) as colorless crystals.
mp186-188°C.
¹H-NMR(CDCl₃) δ: 2.33 (3H, s), 2.38-2.72 (4H, m), 3.2-5.4 (1H, br), 3.73-3.93 (1H, m), 5.55 (1H, s), 7.01 (1H, d, J=8Hz), 7.03 (1H, s), 7.26 (1H, d, J=8Hz).

To a mixture of acetone (80ml), sodium hydroxide (1.2g) and water (80ml) was added dropwise at 0°C a solution of 5-chloro-2-methylbenzaldehyde (4.1g) in acetone (10ml), and the mixture was stirred at the same temperature for 1 hour. Under reduced pressure, acetone was evaporated, and the residue was extracted with ethyl acetate. The organic layer was washed with water and saturated brine, and concentrated under reduced pressure to give 4-(5-chloro-2-methylphenyl)-3-buten-2-one (5.5g) as oil.

To a solution of 20% sodium ethoxide in ethanol (9.5g) was added at room temperature diethyl malonate (4.5g) and then added little by little 4-(5-chloro-2-methylphenyl)-3-buten-2-one (5.5g). The mixture was stirred at room temperature for 30 minutes, refluxed for 2 hours and cooled. The solvent was evaporated, and to the residue was added water. The aqueous layer was washed with ethyl acetate and concentrated. To the residue was added 2M sodium hydroxide (15ml), and the mixture was refluxed for 2 hours and cooled. To the mixture was added 2.5M sulfuric acid (15ml) for 15 minutes, and the mixture was refluxed for 30 minutes and cooled. Precipitated crystals were filtered and washed with water and isopropylether to give 5-(5-chloro-2-methylphenyl)cyclohexane-1,3-dione (2.9g) as colorless crystals.
mp180-181°C.
¹H-NMR(CDCl₃-DMSO-d₆) δ: 2.31 (3H s), 2.35-2.84 (4H, m), 3.37-3.73 (1H, m), 5.56 (1H, s), 6.9-7.43 (1H, br), 7.08-7.26 (3H, m).

### Reference Example 21

A solution of 5-(5-chloro-2-methylphenyl)cyclohexane-1,3-dione (2.9g) and ammonium acetate (2.8g) in ethanol (50ml) was refluxed for 14 hours. Under reduced pressure, the solvent was evaporated, and the residue was dissolved in ethyl acetate, and the mixture was washed with water and saturated brine, and dried with magnesium sulfate. Under reduced pressure, the solvent was evaporated to give 1-amino-5-(5-chloro-2-methylphenyl)cyclohexen-3-one, which was dissolved in a mixture of ethanol (70ml) and toluene (120ml). To the mixture were added 3-oxobutylaldehydedimethylacetal (4.1g) and powdery potassium hydroxide (0.57g), and the mixture was refluxed. To the mixture was added powdery potassium hydroxide (0.14g) 30 minutes later; powdery potassium hydroxide (0.14g) and 3-oxobutylaldehydedimethylacetal (0.33g) 1 hour later; and powdery potassium hydroxide (0.14g) 1.5 hours later, and then the mixture was stirred at the same temperature for 2 hours and cooled. Under reduced pressure, the solvent was evaporated, and to the residue was added ethyl acetate. The organic layer was washed with water and saturated brine, and dried with magnesium sulfate. Under reduced pressure ethyl acetate was evaporated, and the residue was subjected to silica gel column (ethyl acetate-hexane) to give crystals, which were recrystallised from diisopropylether to give 7-(5-chloro-2-methylphenyl)-4-methyl-5,6,7,8-tetrahydroquinoline-5-one (1.1g).
mp125-127°C.
¹H-NMR(CDCl₃) δ: 2.33 (3H, s), 2.72 (3H, s), 2.82-2.96 (2H, m), 3.16-3.46 (2H, m), 3.55-3.74 (1H, m), 7.08-7.33 (4H, m), 8.50 (1H, d, J=5Hz).

### Reference Example 22

To a solution of 7-(5-chloro-2-methylphenyl)-4-methyl-5,6,7,8-tetrahydroquinoline-5-one (1.0g) and aminoguanidine hydrochloride (0.46g) in ethanol (30ml) were added concentrated hydrochloric acid (0.9ml) and water (0.9ml), and the mixture was refluxed for 5 hours. Under reduced pressure, the solvent was evaporated, and the residue was dissolved in water. The mixture was washed with ethyl acetate, and to the aqueous layer was added sodium hydrogen carbonate solution to make it alkaline. The solution was extracted with ethyl acetate, and the organic layer was washed with water and saturated brine, and dried with magnesium sulfate. Under reduced pressure, the solution was concentrated, and the residue was dissolved in ethanol. To the solution was added 1N hydrochloric acid (10ml), and the mixture was concentrated. Precipitated crystals were recrystallised from ethanol to give 7-(5-chloro-2-methylphenyl)-5-guanidinoimino-4-methyl-5,6,7,8-tetrahydroquinoline hydrochloride (1.4g) as colorless crystals.
mp215-220°C.

| Elemental Analysis for C₁₈H₂₀N₅Cl·2HCl·0.3H₂O | | | |
|---|---|---|---|
| Calcd. | C,51.45; | H,5.42; | N,16.67. |
| Found | C,51.49; | H,5.57; | N,16.44. |

¹H-NMR(DMSO-d₆) δ: 2.32 (3H, s), 2.68-3.03 (1H, m), 2.87 (3H, s), 3.13-3.65 (4H, m), 7.12-7.38 (2H, m), 7.54 (1H, s), 7.6-8.45 (4H, br), 7.87 (1H, d, J=6Hz), 8.66 (1H, d, J=6Hz), 11.48 (1H, s).

### Synthesis of (±)-7-(5-fluoro-2-methylphenyl)-5-guanidinoimino-4-methyl-5,6,7,8-tetrahydroquinoline [Compound 7] and (±)-7-(5-fluoro-2-methylphenyl)-5-guanidinoimino-4-methyl-5,6,7,8-tetrahydrocinnoline [Compound 8]

### Reference Example 23

To a solution of 2-bromo-4-fluorotoluene (16.0g) in anhydrous tetrahydrofuran was added dropwise at -78°C a solution of 1.6M butyllithium in hexane (55.5ml), and the mixture was stirred at the same temperature for 30 minutes. To the mixture was added dropwise a solution of dimethylformamide (6.8g) in tetrahydrofuran (20ml), and the mixture was allowed to stand to warm up to 0°C. The reaction solution was poured into ice-water, and the reaction solution was extracted with ethyl acetate. The organic layer was washed with water and saturated brine and dried with magnesium sulfate. Under reduced pressure, the solvent was evaporated to give oil of 5-fluoro-2-methylbenzaldehyde (11.5g).

To a mixture of acetone (80ml), sodium hydroxide (3.7g) and water (100ml) was added dropwise at room temperature a solution of 5-fluoro-2-methylbenzaldehyde (11.5g) in acetone (30ml), and the mixture was stirred at the same temperature for 1 hour. Under reduced pressure, acetone was evaporated, and the residue was extracted with ethyl acetate. The organic layer was washed with water and saturated brine, and concentrated under reduced pressure to give 4-(5-fluoro-2-methylphenyl)-3-buten-2-one (13.4g).

To a solution of 20% sodium ethoxide in ethanol(5.9g) was added at room temperature diethyl malonate (14.0g), and then added little by little 4-(5-fluoro-2-methylphenyl)-3-buten-2-one (13.4g), and the mixture was stirred at room temperature for 30 minutes and then for 2 hours while heating, and cooled. The solvent was evaporated, and to the residue was added water. The aqueous layer was washed with ethyl acetate and concentrated. To the residue was added 2M sodium hydroxide (46ml), and the mixture was refluxed for 2 hours and cooled. To the mixture was added 2.5M sulfuric acid (46ml) for 10 minutes, and the mixture was refluxed for 30 minutes and cooled. Precipitated crystals were filtered and washed with water and isopropylether to give 5-(5-fluoro-2-methylphenyl)cyclohexane-1,3-dione (8.6g) as colorless crystals.
mp175-176°C.
¹H-NMR(CDCl₃) δ: 2.30 (3H, s), 2.27-2.56 (4H, m), 2.5-4.3 (1H, br), 3.44-3.63 (1H, m), 5.55 (1H, s), 6.77-7.01 (2H, m), 7.09-7.17 (1H, m).

### Reference Example 24

A solution of 5-(5-fluoro-2-methylphenyl)cyclohexane-1,3-dione (3.0g) and ammonium acetate (3.1g) in ethanol (50ml) was refluxed for 14 hours. Under reduced pressure, the solvent was evaporated, and the residue was dissolved in ethyl acetate. The mixture was washed with water and saturated brine, and dried with magnesium sulfate. Under reduced pressure, the solvent was evaporated to give 1-amino-5-(5-fluoro-2-methylphenyl)cyclohexen-3-one, which was dissolved in ethanol (70ml) and toluene (120ml). To the mixture were added 3-oxobutylaldehydedimethylacetal (4.1g) and powdery potassium hydroxide (0.57g), and the mixture was refluxed. To the mixture was added powdery potassium hydroxide (0.14g) 30 minutes later; powdery potassium hydroxide (0.14g) and 3-oxobutylaldehydedimethylacetal (0.33g) 1 hour later; and powdery potassium hydroxide (0.14g) 1.5 hours later. Then, the mixture was stirred at the same temperature for 2 hours and cooled. Under reduced pressure, the solvent was evaporated, and to the residue was added ethylacetate. The organic layer was washed with water and saturated brine, and dried with magnesium sulfate. Under reduced pressure, ethyl acetate was evaporated, and the residue was subjected to silica gel column (ethyl acetate-hexane) to give crystals, which were recrystallized from ethyl acetate-hexane to give 7-(5-fluoro-2-methylphenyl)-4-methyl-5,6,7,8-tetrahydroquinoline-5-one (1.5g).
mp113-114°C.
¹H-NMR(CDCl₃) δ: 2.33 (3H, s), 2.71 (3H, s), 2.78-2.98 (2H, m), 3.24 (1H, dd, J=11, 16Hz), 3.28-3.44 (1H, m), 3.55-3.74 (1H, m), 6.82-7.04 (2H, m), 7.12 (1H, d, J=5Hz), 7.07-7.22 (2H, m), 8.50 (1H, d, J=5Hz).

### Reference Example 25

To a solution of 7-(5-fluoro-2-methylphenyl)-4-methyl-5,6,7,8-tetrahydroguinoline-5-one (1.1g) and aminoguanidine hydrochloride (0.54g) in ethanol (30ml) were added concentrated hydrochloric acid (1.0ml) and water (1.0ml), and the mixture was refluxed for 6 hours. Under reduced pressure, the solvent was evaporated, and the residue was dissolved in water. The mixture was washed with ethyl acetate, and to the aqueous layer was added sodium hydrogen carbonate solution to make it alkaline. The solution was extracted with ethyl acetate, and the organic layer was washed with water and saturated brine, dried with magnesium sulfate and concentrated under reduced pressure. The residue was dissolved in ethanol, and to the mixture was added 1N hydrochloric acid (10ml). The mixture was concentrated, and precipitated crystals were recrystallized from ethanol to give 7-(5-fluoro-2-methylphenyl)-5-guanidinoimino-4-methyl-5,6,7,8-tetrahydroquinoline hydrochloride (1.4g) as colorless crystals.
mp202-205°C.

| Elemental Analysis for C₁₈H₂₀N₅F·2HCl·0.5H₂O | | | |
|---|---|---|---|
| Calcd. | C,53.08; | H,5.69; | N,17.19. |
| Found | C,53.33; | H,5.87; | N,16.94. |

¹H-NMR(DMSO-d₆) δ:2.31 (3H, s), 2.72-3.03 (1H, m), 2.90 (3H, s), 3.13-3.57 (4H, m), 6.93-7.06 (1H, m), 7.17-7.4 (2H, m), 7.5-8.4 (4H, br), 7.85 (1H, d, J=6Hz), 8.65 (1H, d, J=6Hz), 11.39 (1H, s).

### Reference Example 26

A mixture of 5-(5-fluoro-2-methylphenyl)cyclohexane-1,3-dione (2.2g), p-toluenesulfonylhydrazide (2g) and ethanol (20ml) was refluxed for 2.5 hours and cooled, and precipitated crystals were filtered and washed with ethanol to give 5-(3-fluoro-6-methylphenyl)-1-[2-(4-methylphenylsulfonyl)hydrazino]cyclohexane-3-one (1.64g) as colorless crystals.
mp241-242°C(decomp.).
¹H-NMR(DMSO-d₆) δ:2.15 (1H, dd), 2.24 (3H, s), 2.27-2.60 (6H, m), 3.20-3.37 (1H, m), 5.23 (1H, s), 6.88-6.98 (1H, m), 7.12-7.22 (2H, m), 7.42 (2H, d) 7.72 (2H, d), 8.73 (1H, br), 9.82 (1H, br).

### Reference Example 27

To a mixture of 5-(5-fluoro-2-methylphenyl)-1-[2-(4-methylphenylsulfonyl)hydrazino]cyclohexane-3-one (1.63g), anhydrous potassium carbonate (1.45g), methanol (20ml) and 1,2-dimethoxyethane (10ml) was added under ice-cooling 1-bromopropan-2-one (0.75g), and the mixture was stirred at room temperature for 2 hours and then at 80°C for 5 hours. Under reduced pressure, the solvent was evaporated, and to the residue were added ethyl acetate (70ml) and water (30ml). The mixture was shaken, and the separated upper layer was washed with water and concentrated under reduced pressure. The residue was purified with silica gel column chromatography to give 7-(5-fluoro-2-methylphenyl)-4-methyl-5,6,7,8-tetrahydrocinnoline-5-one (0.265g) as yellow brown crystals.
mp127-128°C.
¹H-NMR(CDCl₃) δ: 2.34 (3H, s), 2.71 (3H, s), 2.85 (1H, dd), 2.98 (1H, ddd), 3.36 (1H, dd), 3.62-3.77 (2H, m), 6.87-7.02 (2H, m), 7.20 (1H, dd), 9.16(1H,s).

### Reference Example 28

To a solution of 7-(5-fluoro-2-methylphenyl)-4-methyl-5,6,7,8-tetrahydrocinnoline-5-one (0.265g) and aminoguanidine hydrochloride (0.12g) in ethanol (3ml) was added concentrated hydrochloric acid (0.15ml), and the mixture was stirred at 110°C (bath temperature) for 3.5 hours. The reaction solution was cooled to room temperature, and the crystals were filtered and dried to give 7-(5-fluoro-2-methylphenyl)-5-guanidinoimino-4-methyl-5,6,7,8-tetrahydrocinnoline hydrochloride (0.33g) as blue gray crystals.
mp240°C(decomp.).
¹H-NMR(DMSO-d₆) δ: 2.30 (3H, s), 2.73-2.90 (4H, m), 3.17-3.50 (4H, m), 6.96-7.06 (1H, m), 7.21-7.36 (2H, m), 8.02 (4H, br), 9.35 (1H, s), 11.66 (1H, s).

### Synthesis of (±)-7-(2-chloro-5-fluorophenyl)-5-guanidinoimino-4-methyl-5,6,7,8-tetrahydroquinoline [Compound 9]

### Reference Example 29

To a solution of 2-chloro-5-fluorotoluene (5.0g) in anhydrous acetic acid (40ml) was added dropwise under ice-cooling concentrated sulfuric acid (40ml), and then added dropwise a solution of anhydrous chromic acid (9.3g) in anhydrous acetic acid (40ml) for 2 hours. The mixture was stirred at the same temperature for 1 hour, poured into ice-water and extracted with diethylether. The organic layer was washed with sodium carbonate solution, water and saturated brine and dried with magnesium sulfate. Under reduced pressure, the solvent was evaporated, and the residue was dissolved in tetrahydrofuran (10ml). To the mixture were added water (4ml) and concentrated sulfuric acid (4ml), and the mixture was stirred at 100°C for 30 minutes and cooled. The reaction solution was extracted with ethyl acetate. The organic layer was washed with sodium carbonate solution, water and saturated brine and dried with magnesium sulfate. Under reduced pressure, the solvent was evaporated to give the residue, which was subjected to silica gel column chromatography to give 2-chloro-5-fluorobenzaldehyde (1.6g).

The similar reaction was repeated to give 2-chloro-5-fluorobenzaldehyde (1.2g).

In water (55ml) was dissolved sodium hydroxide (0.78g), and to the mixture was added acetone (55ml), and then added dropwise a solution of 2-chloro-5-fluorobenzaldehyde (2.8g) in acetone (10ml). The reaction solution was stirred at room temperature for 2 hours, and acetone was evaporated under reduced pressure. The residue was extracted with ethyl acetate, and the organic layer was washed with water and saturated brine, and concentrated under reduced pressure to give 4-(2-chloro-5-fluorophenyl)-3-buten-2-one (0.24g).

To a solution of 20% sodium ethoxide in ethanol (0.43g) was added at room temperature diethyl malonate (0.2g), and then added little by little 4-(2-chloro-5-fluorophenyl)-3-buten-2-one (0.24g). The mixture was stirred at room temperature for 30 minutes, refluxed for 2 hours and cooled, and the solvent was evaporated. The residue was dissolved in water, and the aqueous layer was washed with ethyl acetate and concentrated. To the reside was added 2M sodium hydroxide (0.7ml), and the mixture was refluxed for 2 hours cooled. To the mixture was added 2.5M sulfuric acid (0.7ml), and the mixture was refluxed for 15 minutes. The mixture was extracted with ethyl acetate, and the organic layer was washed with water and saturated brine and dried with magnesium sulfate. Under reduced pressure, the solvent was evaporated to give 5-(2-chloro-5-fluorophenyl)cyclohexane-1,3-dione (0.17g) as oil.

A solution of 5-(5-chloro-2-fluorophenyl)cyclohexane-1,3-dione (0.17g) and ammonium acetate (0.16g) in ethanol (10ml) was refluxed for 12 hours. Under reduced pressure, the solvent was evaporated, and to the residue was added ethyl acetate. The organic layer was washed with sodium carbonate solution, water and saturated brine and dried with magnesium sulfate. Under reduced pressure, the solvent was evaporated, and the residue was dissolved in ethanol (3.5ml) and toluene (6ml). To the mixture were added 3-oxobutylaldehydedimethylacetal (0.21g) and powdery potassium hydroxide (34mg), and the mixture was refluxed for. To the mixture was added powdery potassium hydroxide (0.07g) 30 minutes later; powdery potassium hydroxide (0.07g) and 3-oxobutylaldehydedimethylacetal (17mg) 1 hour later; and powdery potassium hydroxide (0.07g) 1.5 hours later. Then, the mixture was stirred at the same temperature for 2 hours and cooled. Under reduced pressure, the solvent was evaporated, and the residue was extracted with ethyl acetate. The organic layer was washed with water and saturated brine and dried with magnesium sulfate. Under reduced pressure, ethyl acetate was evaporated, and the residue was subjected to silica gel column chromatography(ethyl acetate-hexane) to give 7-(2-chloro-5-fluorophenyl)-4-methyl-5,6,7,8-tetrahydroquinoline-5-one.

To a solution of 7-(2-chloro-5-fluorophenyl)-4-methyl-5,6,7,8-tetrahydroquinoline-5-one in ethanol (10ml) were added aminoguanidine hydrochloride (0.041g), concentrated hydrochloric acid (0.078ml) and water (0.078ml), and the mixture was refluxed for 4 hours. Under reduced pressure, the solvent was evaporated, and to the residue was added water. The aqueous layer was washed with ethyl acetate, and to the aqueous layer was added sodium hydrogen carbonate solution to make it alkaline. The solution was extracted with ethyl acetate, and the organic layer was washed with water and saturated brine, dried with magnesium sulfate, and concentrated under reduced pressure. The residue was dissolved in 1N hydrochloric acid (1ml) and concentrated to give crystals, which were recrystallized from ethanol-ethyl acetate to give 7-(2-chloro-5-fluorophenyl)-5-guanidinoimino-4-methyl-5,6,7,8-tetrahydroquinoline hydrochloride (0.05g) as colorless crystals.
mp. 268°C(decomp.).
¹H-NMR(DMSO-d₆) δ: 2.76-3.05 (1H, m), 2.84 (3H, s), 3.13-3.75 (4H, m), 7.0-8.4 (4H, br), 7.2-7.34 (1H, m), 7.52-7.66 (2H, m), 7.76 (1H, d, J=6Hz), 8.6 (1H, d, J=6Hz), 11.36 (1H, s).

### Synthesis of (±)-7-(5-chloro-2-fluorophenyl)-5-guanidinoimino-4-methyl-5,6,7,8-tetrahydroquinoline [Compound 10] and (±)-7-(5-chloro-2-fluorophenyl)-5-guanidinoimino-4-methyl-5,6,7,8-tetrahydrocinnoline [Compound 11]

### Reference Example 30

To a solution of 1.6M butyllithium in hexane (32ml) was added anhydrous tetrahydrofuran (100ml), and then added dropwise 2,2,6,6-tetramethylpiperidine (8.4ml) at -70°C or less. To the mixture was added dropwise a solution of 1-chloro-4-fluorobenzene (6.5g) in anhydrous tetrahydrofuran (20ml), and the mixture was stirred at -70°C or less for 2 hours. To the mixture was added dropwise a solution of dimethylformamide (6.5g) in tetrahydrofuran (15ml), and the mixture was allowed to stand to warm up to 0°C. The reaction solution was poured into ice-water and extracted with ethyl acetate. The organic layer was washed with water and saturated brine, and dried with magnesium sulfate. Under reduced pressure, the solvent was evaporated to give oil of 5-chloro-2-fluorobenzaldehyde (5.1g).

To a mixture of acetone (22ml), sodium hydroxide (0.83g) and water (55ml) was added dropwise at room temperature a solution of 5-chloro-2-fluorobenzaldehyde (3.5g) in acetone (20ml), and the mixture was stirred at the same temperature for 15 hours. Under reduced pressure, acetone was evaporated, and the residue was extracted with ethyl acetate. The organic layer was washed with water and saturated brine, and concentrated under reduced pressure to give 4-(5-chloro-2-fluorophenyl)-3-buten-2-one (4.3g).

To a solution of 20% sodium ethoxide in ethanol(7.4g) was added at room temperature diethyl malonate (3.6g), and then added little by little 4-(5-chloro-2-fluorophenyl)-3-buten-2-one (4.3g). The mixture was stirred at room temperature for 30 minutes and then for 2 hours, and cooled. The solvent was evaporated, and to the residue was added water. The aqueous layer was washed with ethyl acetate and concentrated. To the residue was added 2M sodium hydroxide (17ml), and the mixture was refluxed for 1 hour and cooled. To the mixture was added 2.5M sulfuric acid (17ml) for 10 minutes, and the mixture was refluxed for 30 minutes and cooled. Precipitated crystals were filtered and washed with water and isopropylether to give 5-(5-chloro-2-fluorophenyl)cyclohexane-1,3-dione (4.1g) as pale yellow crystals.
mp176-177°C.
¹H-NMR(DMSO-d₆) δ: 2.33-2.72 (4H, m), 3.43-3.65 (1H, m), 5.30 (1H, s), 7.18-7.39 (2H, m), 7.47-7.52 (1H, m), 10.78 (1H, br).

### Reference Example 31

A solution of 5-(5-chloro-2-fluorophenyl)cyclohexane-1,3-dione (2g) and ammonium acetate (1.9g) in ethanol (15ml) was refluxed for 15 hours. The reaction solution was concentrated under reduced pressure, and to the residue were added water (30ml) and ethyl acetate (150ml). The mixture was shaken, and the separated upper layer was washed with water and concentrated under reduced pressure. The residue was washed with ethyl acetate and dried to give 1-amino-5-(5-chloro-2-fluorophenyl)cyclohexane-3-one (1.45g) as yellow crystals.
mp233-234°C.
¹H-NMR(DMSO-d₆) δ:2.20 (1H, dd), 2.34-2.53 (2H, m), 2.64 (1H, dd), 3.39-3.55 (1H, m), 5.02 (1H, s), 6.88 (1H, br), 7.19-7.50 (3H, m).

### Reference Example 32

To a mixture of 1-amino-5-(5-chloro-2-fluorophenyl)cyclohexane-3-one (1.33g), 1,1-dimethoxy-3-butanone (2ml), toluene (20ml) and ethanol (9ml) was added granulated potassium hydroxide (0.4g) at 110-115°C while stirring. Just after potassium hydroxide was dissolved, granulated potassium hydroxide (0.3g) was added to the mixture. With 30 minutes intervals, granulated potassium hydroxide (0.1g) was added three times to the mixture. To the mixture was added 1,1-dimethoxy-3-butanone (1ml) 2 hours after the reaction had started, and then granulated potassium hydroxide (0.1g) was added to the mixture. The mixture was stirred for 1 hour under the same conditions. The reaction solution was concentrated under reduced pressure, and to the residue was added water (30ml) and ethyl acetate (100ml). The mixture was shaken and the separated upper layer was washed with water. To the mixture was added concentrated hydrochloric acid (0.5ml). The mixture was concentrated under reduced pressure, and the residue washed with a little amount of ethanol and dried to give 7-(5-chloro-2-fluorophenyl)-4-methyl-5,6,7,8-tetrahydroquinoline-5-one hydrochloride (1.05g).
mp179-180°C.
¹H-NMR(DMSO-d₆) δ:2.79-2.90(4H,m),3.16(1H,dd),3.43-3.69(2H,m),3.82-3.98(1H,m),7.26-7.47(2H,m),7.59(1H,dd),7.75(1H,d),8.77(1H,d).

### Reference Example 33

To a solution of 7-(5-chloro-2-fluorophenyl)-4-methyl-5,6,7,8-tetrahydroquinoline-5-one hydrochloride (0.6g) and aminoguanidine hydrochloride (0.233g) in ethanol (10ml) was added concentrated hydrochloric acid (0.1ml), and the mixture was stirred at 110°C (bath temperature) for 2 hours. The reaction solution was cooled to room temperature, and the crystals were filtered and dried to give 7-(5-chloro-2-fluorophenyl)-5-guanidinoimino-4-methyl-5,6,7,8-tetrahydroquinoline hydrochloride (0.6g) as colorless crystals.
mp 300°C or more.

| Elemental Analysis for C₁₆H₁₆N₆ClF·2HCl | | | |
|---|---|---|---|
| Calcd. | C,45.79; | H,4.32; | N,20.02. |
| Found | C,45.74; | H,4.36; | N,19.88. |

¹H-NMR(DMSO-d₆) δ: 2.88 (3H, s), 2.95 (1H, dd), 3.23 (1H, dd), 3.39-3.70 (3H, m), 7.26-7.48 (2H, m), 7.70 (1H, dd), 7.85 (1H, d), 7.96 (4H, br), 8.63 (1H, d) 11.60 (1H, s).

### Reference Example 34

A mixture of 5-(5-chloro-2-fluorophenyl)cyclohexane-1,3-dione (2.04g), p-toluenesulfonylhydrazide (1.67g) and ethanol (15ml) was refluxed for 2.5 hours and cooled, and precipitated crystals were filtered and washed with ethanol to give 5-(5-chloro-2-fluorophenyl)-1-[2-(4-methylphenylsulfonyl)hydrazino]cyclohexane-3-one (2.08g) as colorless crystals.
mp244-245°C(decomp.).
¹H-NMR(DMSO-d₆) δ: 2.23 (1H, dd), 2.40 (3H, s), 2.43-2.60 (3H, m), 3.32-3.47 (1H, m), 5.23 (1H, s), 7.19-7.49 (5H, m), 7.72 (2H, d), 8.79 (1H, br), 9.85 (1H, s).

### Reference Example 35

To a mixture of 5-(5-chloro-2-fluorophenyl)-1-[2-(4-methylphenylsulfonyl)hydrazino]cyclohexane-3-one (2.0g), anhydrous potassium carbonate (1.68g), methanol (20ml) and 1,2-dimethoxyethane (10ml) was added under ice-cooling 1-bromopropan-2-one (0.87g), and the mixture was stirred for 2 hours at room temperature, and then for 6 hours at 80°C. Under reduced pressure, the solvent was evaporated, and to the residue was added ethyl acetate (70ml) and water (30ml). The mixture was shaken, and the separated upper layer was washed with water and concentrated under reduced pressure. The residue was purified with silica gel column chromatography to give 7-(5-chloro-2-fluorophenyl)-4-methyl-5,6,7,8-tetrahydrocinnoline-5-one (0.338g) as yellow brown crystals.
mp124-125°C (recrystallized from ethyl acetate-hexane).
¹H-NMR(CDCl₃) δ: 2.71 (3H, s), 2.93 (1H, dd), 3.05 (1H, ddd), 3.48 (1H, dd), 3.68-3.85 (2H, m), 7.02-7.10 (1H, m), 7.24-7.31 (2H, m), 9.16 (1H, s).

### Reference Example 36

To a mixture of 7-(5-chloro-2-fluorophenyl)-4-methyl-5,6,7,8-tetrahydrocinnoline-5-one (0.33g) and aminoguanidine hydrochloride (0.133g) in ethanol (4ml) was added concentrated hydrochloric acid (0.1ml), and the mixture was stirred at 110°C (bath) for 2 hours. The reaction solution was cooled to room temperature, and the crystals were filtered and dried to give 7-(5-chloro-2-fluorophenyl)-5-guanidinoimino-4-methyl-5,6,7,8-tetrahydrocinnoline hydrochloride (0.345g) as blue gray crystals.
mp 300°C or more.

| Elemental Analysis for C₁₆H₁₆N₆ClF·2HCl | | | |
|---|---|---|---|
| Calcd. | C,45.79; | H,4.32; | N,20.02. |
| Found | C,45.74; | H,4.36; | N,19.88. |

¹H-NMR(DMSO-d₆) δ: 2.77 (3H, s), 2.96 (1H, dd), 3.17-3.60 (4H, m), 7.27-7.48 (2H, m), 7.71 (1H, dd), 8.03 (4H, br), 9.33 (1H, s), 11.78 (1H, br).

### Effect of the Invention

The pharmaceutical composition of the present invention formulated by combination of a compound having endothelin antagonistic activity or a salt thereof with a compound having activity for inhibiting Na-H exchange or a salt thereof serves to decrease remarkably the dosage of the individual effective components, and as a result, suppresses undesirable side effects observed in the case of administering the respective compounds singly, and can advantageously used as a prophylactic or therapeutic agent of ischemic disease, especially, ischemic cardiac disease, and in particular, myocardial infarction, cardiac insufficiency, arrhythmia, etc.

## Claims

1. A pharmaceutical composition for preventing or treating ischemic disease comprising a compound having endothelin antagonistic activity or a salt thereof in combination with a compound having activity for inhibiting Na-H exchange or a salt thereof.

2. A composition according to claim 1, which is for the prevention or treatment of ischemic heart disease.

3. A composition according to claim 1, which is for the prevention or treatment of ischemic encephalic disease.

4. A composition according to claim 1, which is for the prevention or treatment of ischemic renal disease.

5. A composition according to claim 1, which is for the prevention or treatment of myocardial infarction.

6. A composition according to claim 1, which is for the prevention or treatment of arrhythmia.

7. A composition according to claim 1, which is for the prevention or treatment of congestive heart failure.

8. A composition according to claim 1, wherein the compound having endothelin antagonistic activity or a salt thereof is Cyclo[-D-Asp-Asp(R1)-Asp-D-Thg(2)-Leu-D-Trp-], in which Asp(R1) is aspartic acid β-4-phenylpiperazinamide residue and Thg(2) is 2-thienylglycine residue, or a salt thereof.

9. A composition according to claim 1, wherein the compound having activity for inhibiting Na-H exchange or a salt thereof is cariporide.

10. A composition according to claim 1, wherein the compound having activity for inhibiting Na-H exchange or a salt thereof is a compound of the formula: 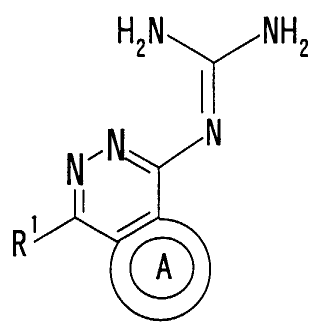 wherein the ring A is an optionally substituted benzene ring or an optionally substituted nitrogen-containing 6-membered aromatic ring and R¹ is an optionally substituted aromatic ring group, or a salt thereof.

11. A composition according to claim 1, wherein the compound having activity for inhibiting Na-H exchange or a salt thereof is a compound of the formula: 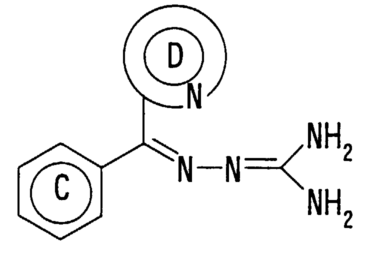 wherein the ring C is an optionally substituted benzene ring, and the ring D is an optionally substituted nitrogen-containing 6-membered aromatic ring, or a salt thereof.

12. A composition according to claim 1, wherein the compound having activity for inhibiting Na-H exchange or a salt thereof is a compound of the formula: 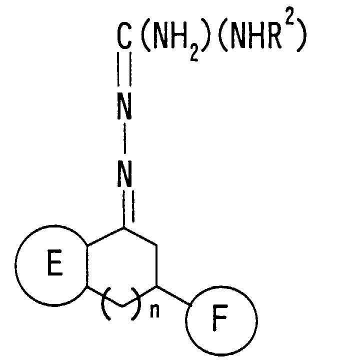 wherein the ring E is an optionally substituted 5- to 6-membered aromatic heterocyclic ring, the ring F is an optionally substituted 5- to 6-membered aromatic homocyclic ring or an optionally substituted 5- to 6-membered aromatic heterocyclic ring, R² is a hydrogen atom, a hydroxy group or a lower alkyl group and n is 0 or 1, or a salt thereof.

13. A pharmaceutical composition for preventing or treating ischemic disease comprising disodium salt of Cyclo[-D-Asp-Asp(R1)-Asp-D-Thg(2)-Leu-D-Trp-], in which Asp(R1) is aspartic acid β-4-phenylpiperazinamide residue and Thg(2) is 2-thienylglycine residue, in combination with cariporide.

14. Use of a compound having endothelin antagonistic activity or a salt thereof in combination with a compound having activity for inhibiting Na-H exchange or a salt thereof, for the manufacture of a medicament for preventing or treating ischemic disease.

15. A pharmaceutically effective combination for preventing or treating ischemic disease, comprising an administration of a compound having endothelin antagonistic activity or a salt thereof with a compound having activity for inhibiting Na-H exchange or a salt thereof.

16. A pharmaceutical composition for preventing or treating ischemic disease, which is formulated by the combination of a compound having endothelin antagonistic activity or a salt thereof with a compound having activity for inhibiting Na-H exchange or a salt thereof.
